(19) Europäisches Patentamt
European Patent Office
Office européen des brevets

(11) EP 4 023 679 A1

(12) EUROPEAN PATENT APPLICATION
published in accordance with Art. 153(4) EPC

(43) Date of publication:
**06.07.2022 Bulletin 2022/27**

(21) Application number: **20857795.7**

(22) Date of filing: **28.08.2020**

(51) International Patent Classification (IPC):
***C07K 19/00*** (2006.01) ***C07K 14/71*** (2006.01)
***A61K 38/17*** (2006.01) ***A61P 35/00*** (2006.01)
***A61K 39/395*** (2006.01) ***C12N 15/62*** (2006.01)
***C12N 15/63*** (2006.01)

(52) Cooperative Patent Classification (CPC):
**A61K 38/17; A61K 39/395; A61P 35/00; C07K 14/71; C07K 19/00; C12N 15/62; C12N 15/63**

(86) International application number:
**PCT/CN2020/111983**

(87) International publication number:
**WO 2021/037184 (04.03.2021 Gazette 2021/09)**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(30) Priority: **30.08.2019 CN 201910815301**

(71) Applicants:
- **Nanjing Shunxin Pharmaceutical Co., Ltd. of Chiatai Tianqing Pharmaceutical Group**
  **Nanjing, Jiangsu 211100 (CN)**
- **CHIA TAI TIANQING PHARMACEUTICAL GROUP CO., LTD.**
  **Lianyungang, Jiangsu 222062 (CN)**

(72) Inventors:
- **ZHAO, Wei**
  **Lianyungang, Jiangsu 222062 (CN)**
- **LI, Yingchun**
  **Lianyungang, Jiangsu 222062 (CN)**
- **XIE, Lianxiang**
  **Lianyungang, Jiangsu 222062 (CN)**
- **LU, Yamin**
  **Lianyungang, Jiangsu 222062 (CN)**
- **LV, Haili**
  **Lianyungang, Jiangsu 222062 (CN)**
- **DU, Xiuzhen**
  **Lianyungang, Jiangsu 222062 (CN)**
- **XU, Tongjie**
  **Lianyungang, Jiangsu 222062 (CN)**
- **CHENG, Yanju**
  **Lianyungang, Jiangsu 222062 (CN)**
- **ZHANG, Xiquan**
  **Lianyungang, Jiangsu 222062 (CN)**

(74) Representative: **v. Bezold & Partner Patentanwälte - PartG mbB**
**Ridlerstraße 57**
**80339 München (DE)**

# (54) FUSION PROTEIN TARGETING PD-L1 AND TGF-BETA AND USE THEREOF

(57) Provided are a fusion protein targeting PD-L1 and TGF-β and use thereof. The fusion protein is a fusion protein comprising an antibody or antigen-binding fragment that specifically binds to human PD-L1 and a human TGFβRII binding domain. Further provided are a polynucleotide encoding the fusion protein, a host cell containing the polynucleotide, and the use of the fusion protein in the preparation of an anti-tumor drug.

EP 4 023 679 A1

## Description

## FIELD OF THE INVENTION

The present application relates to the field of biotechnology, and specifically to a fusion protein comprising: (a) at least one heavy chain variable domain and at least one light chain variable domain of an antibody binding to human programmed death-ligand 1 (PD-L1); and (b) a human TGF-β RII or a TGF-β binding fragment thereof. The present application further relates to a method for preparing the fusion protein and use thereof (e.g., for cancer treatment).

## BACKGROUND

In recent years, tumors show increasing incidences, along with poor efficacy for malignancies and high metastasis rate in advanced diseases. At present, conventional therapies in the clinical use, such as radiotherapy, chemotherapy and surgery, can relieve the pain and prolong the survival period, but have limitations. Therefore, a method for activating the immune system and reforming the host's immune response by immunotherapy to induce tumor regression and stabilize the disease has gradually become a hot spot in the field of oncotherapy.

Programmed death-ligand 1 (PD-L1), one of the ligands of programmed death receptor 1 (PD-1), is mainly expressed on T cells, B cells, macrophages and dendritic cells, and demonstrates up-regulated expression on activated cells. Binding of PD-L1 and PD-1 forms a receptor-ligand complex before sending inhibitory signals, including signals for inducing the IL-10 (inflammation and immunosuppressive factors) production, down-regulating anti-apoptosis gene bcl-2 to promote the apoptosis of antigen-specific T cells, inhibiting the proliferation of $CD8^+$ T cells in lymph nodes, etc. In addition to the capability of binding to PD-1 with a high affinity, PD-L1 can also bind to CD80 with a low affinity, thereby suppressing T cell activity. Researches show that PD-L1 protein is highly expressed in various human tumor tissues, such as breast cancer, lung cancer, gastric cancer, intestinal cancer, kidney cancer and melanoma, such that tumor cells can evade the attack of the immune system. Meanwhile, the expression level of PD-L1 is closely related to the clinical treatment and prognosis of a subject. This suggests that PD-L1 is a prospective target in tumor immunotherapy.

Transforming growth factor β (TGF-β) is a multifunctional cytokine that plays an important regulatory role in cell proliferation and differentiation, migration and adhesion, extracellular matrix production, angiogenesis and connective tissue formation, immune functions, and other processes. TGF-β has both tumor-suppressing and tumor-promoting effects. However, with the progression of tumors, it promotes the tumor metastasis and immune escape as well as induces tumor angiogenesis by epithelial-mesenchymal transition, ultimately leading to disease progression. TGF-β recognizes transforming growth factor β receptor II (TGF-β RII) and subsequently forms a complex that phosphorylates the TGF-β RII. Then the complex binds to TGF-β RI dimer to form a heterotetrameric receptor complex for signaling. Researches show that inhibition of TGF-β signaling by TGF-β receptors can reduce tumor metastasis. This makes the TGF-β receptor one of the important directions for developing tumor drugs at present.

Inhibitors of PD-L1 and other immune checkpoint proteins act less efficiently. They have long-term effects only on certain subjects, and may lead to fatal autoimmune adverse events. Therefore, how to improve their therapeutic effects and reduce the toxicity remains a focus of research in the field of immunotherapy. However, recent researches showed that TGF-β is the leading cause of the failure of immune checkpoint inhibitor therapies. In general, inhibiting TGF-β signaling pathways, on the basis of inhibiting PD-L1/PD-1 pathways, can effectively improve the anti-tumor efficacy.

## SUMMARY

The purpose of the present application is at least to provide a fusion protein targeting PD-L1 and TGF-β, which may

a) specifically bind to PD-L1 and/or block the binding of PD-1/PD-L1 and a signaling pathway thereof; and/or
b) bind to TGF-β or inhibit TGF-β signaling, so as to reduce its promoting effect on tumor progression.

In another aspect, the present application provides a protein molecule comprising a partial or intact TGF-β RII that can bind to TGF-β, and an antibody or an antigen binding fragment thereof that binds to an immune checkpoint protein (e.g., PD-L1), as an effective anti-tumor and anti-cancer medicament.

In yet another aspect, the present application provides a fusion protein comprising:

(a) an antibody or an antigen binding fragment thereof (e.g., at least one heavy chain variable domain and at least one light chain variable domain) that binds to human programmed death-ligand 1 (PD-L1); and/or

(b) a human TGF-β binding domain.

In certain embodiments, the human TGF-β binding domain is a human TGF-β RII or a TGF-β binding fragment thereof, e.g., a polypeptide or peptide fragment having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 36, or any fragment described herein.

In certain embodiments, a CDR1 sequence of the heavy chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 1 or SEQ ID NO: 15, a CDR2 sequence of the heavy chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 2 or SEQ ID NO: 16, and a CDR3 sequence of the heavy chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 3 or SEQ ID NO: 17; and a CDR1 sequence of the light chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 4 or SEQ ID NO: 18, a CDR2 sequence of the light chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 5 or SEQ ID NO: 19, and a CDR3 sequence of the light chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 6 or SEQ ID NO: 20. In certain embodiments, the heavy chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 7 or SEQ ID NO: 21, and the light chain variable domain has at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 8 or SEQ ID NO: 22.

| | | |
|---|---|---|
| HCDR1: | SYGMS | SEQ ID NO: 1 |
| | TYGVH | SEQ ID NO: 15 |
| HCDR2: | SISSGGSTYYPDSVKG | SEQ ID NO: 2 |
| | VIWRGVTTDYNAAFMS | SEQ ID NO: 16 |
| HCDR3: | GYDSGFAY | SEQ ID NO: 3 |
| | LGFYAMDY | SEQ ID NO: 17 |
| LCDR1: | ASQSVSTSSSSFMH | SEQ ID NO: 4 |
| | KASQSVSNDVA | SEQ ID NO: 18 |
| LCDR2: | YASNLES | SEQ ID NO: 5 |
| | YAANRYT | SEQ ID NO: 19 |
| LCDR3: | QHSWEIPYT | SEQ ID NO: 6 |
| | QQDYTSPYT | SEQ ID NO: 20 |

The fusion protein may further comprise a linker peptide linking a C terminus of the antibody or the antigen binding fragment thereof to an N terminus of the TGF-β binding domain. In certain embodiments, the linker peptide may be a flexible linker peptide or a rigid linker peptide, and optionally is a combination of glycine and serine. For example, the linker peptide is $(G_4S)_xG$, wherein x is optionally an integer of 3-6, preferably 4-5, and most preferably 4. Linkage without using a linker peptide can also be employed.

In certain embodiments, the fusion protein comprises at least one light chain variable domain and at least one heavy chain variable domain of an antibody. When combined, the light chain variable domain and the heavy chain variable domain form an antigen binding site that binds to human PD-L1.

In certain embodiments, the fusion protein may comprise a constant region of an immunoglobulin, or a fragment, analog, variant or derivative of the constant region. In some embodiments, the constant region is derived from a human immunoglobulin heavy chain, e.g., a heavy chain of IgG1, IgG2, IgG3 and IgG4, or other types of immunoglobulins, and preferably a heavy chain of IgG1. In some embodiments, the constant region may comprise any modification described herein, e.g., insertion, deletion, substitution, or chemical modification of amino acids. In some embodiments, the constant region comprises a mutation that alters the effector function. For example, a lysine residue at the C terminus of the antibody constant region is mutated to a hydrophobic amino acid, such as alanine or leucine, thereby reducing hydrolytic cleavage by proteases and prolonging the serum half-life. In some embodiments, any amino acid residue of the constant region may be substituted by an amino acid residue of any allotype, preferably by an amino acid residue of G1m(3) and/or nG1m(1).

In certain embodiments, the fusion protein comprises an antibody or an antigen binding fragment thereof that binds to PD-L1, and a TGF-β binding domain. The antibody or the antigen binding fragment thereof may optionally comprise a modified (e.g., any modification described herein) constant region, comprising a mutation of K at the C terminus of the constant region to A, or a substitution by an amino acid of an allotype. The TGF-β binding domain comprises a human TGF-β RII or a fragment or variant thereof that can bind to TGF-β, or an extracellular domain of human TGF-β RII.

In certain embodiments, the fusion protein comprises (a) a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to a heavy chain variable domain amino acid sequence set forth in SEQ ID NO: 7 and a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to a light chain variable domain amino acid sequence set forth in SEQ ID NO: 8, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to a heavy chain variable domain amino acid sequence set forth in SEQ ID NO: 21 and a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to a light chain variable domain amino acid sequence set forth in SEQ ID NO: 22; and (b) a TGF-β binding domain. In some certain embodiments, the TGF-β binding domain is a human TGF-β RII or a TGF-β binding fragment or a variant thereof, or a polypeptide or peptide fragment having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 36:

EVQLVESGGGLVKPGGSLRLSCAASGFIFRSYGMSWVRQAPGKGLEWVASISSGGSTYYPDSVKGRFTIS RDNAKNSLYLQMNSLRAEDTAVYDCARGYDSGFAYWGQGTLVTVSS (SEQ ID NO: 7);

DIVLTQSPASLAVSPGQRATITCRASQSVSTSSSSFMHWYQQKPGQPPKLLIKYASNLESGVPARFSGSGSG TDFTLTINPVEANDTANYYCQHSWEIPYTFGQGTKLEIK (SEQ ID NO: 8);

QITLKESGPTLVKPTQTLTLTCTVSGFSLSTYGVHWIRQPPGKALEWLGVIWRGVTTDYNAAFMSRLTITK DNSKNQVVLTMNNMDPVDTATYYCARLGFYAMDYWGQGTLVTVSS (SEQ ID NO:21);

DIQMTQSPSSLSASVGDRVTITCKASQSVSNDVAWYQQKPGKAPKLLIYYAANRYTGVPDRFSGSGYGTD FTFTISSLQPEDIATYFCQQDYTSPYTFGQGTKLEIK (SEQ ID NO: 22);

IPPHVQKSVNNDMIVTDNNGAVKFPQLCKFCDVRFSTCDNQKSCMSNCSITSICEKPQEVCVAVWRKND ENITLETVCHDPKLPYHDFILEDAASPKCIMKEKKKPGETFFMCSCSSDECNDNIIFSEEYNTSNPD (SEQ ID NO: 36).

In certain embodiments, the fusion protein comprises (a) a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to a heavy chain amino acid sequence set forth in SEQ ID NO: 9 and a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to a light chain amino acid sequence set forth in SEQ ID NO: 10, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to a heavy chain amino acid sequence set forth in SEQ ID NO: 23 and a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to a light chain amino acid sequence set forth in SEQ ID NO: 24; and (b) a TGF-β binding domain. In certain embodiments, the fusion protein comprises (a) a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to a heavy chain amino acid sequence set forth in SEQ ID NO: 29 and a sequence having at least 80%, 81%, 82%, 83%, 84%, 85%, 86%, 87%, 88%, 89%, 90%, 91%, 92%, 93%, 94%, 95%, 96%, 97%, 98%, 99%, or 100% identity to a light chain amino acid sequence set forth in SEQ ID NO: 10, or a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to a heavy chain amino acid sequence set forth in SEQ ID NO: 32 and a sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to a light chain amino acid sequence set forth in SEQ ID NO: 24; and (b) a TGF-β binding domain. In some certain embodiments, the TGF-β binding domain is a human TGF-β RII or a TGF-β binding fragment or a variant thereof, or a polypeptide or peptide fragment having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 36.

In some embodiments, the fusion protein comprises any antigen binding protein described in the art that specifically binds to PD-L1 or an antigen binding fragment thereof. Preferably, the antigen binding protein that specifically binds to PD-L1 comprises the following amino acid sequences: a heavy chain CDR1 having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 1 or SEQ ID NO: 15; a heavy chain CDR2 having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 2 or SEQ ID NO: 16; a heavy chain CDR3 having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 3 or SEQ ID NO: 17; a light chain CDR1 having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 4 or SEQ ID NO: 18; a light chain CDR2 having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 5 or SEQ ID NO: 19; and a light chain CDR3 having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 6 or SEQ ID NO: 20.

In some embodiments, the antigen binding protein that specifically binds to PD-L1 comprises the following amino acid sequences: a heavy chain CDR1 selected from SEQ ID NO: 1 and SEQ ID NO: 15; a heavy chain CDR2 selected from SEQ ID NO: 2 and SEQ ID NO: 16; a heavy chain CDR3 selected from SEQ ID NO: 3 and SEQ ID NO: 17; a light chain CDR1 selected from SEQ ID NO: 4 and SEQ ID NO: 18; a light chain CDR2 selected from SEQ ID NO: 5 and SEQ ID NO: 19; and a light chain CDR3 selected from SEQ ID NO: 6 and SEQ ID NO: 20.

In some embodiments, the antigen binding protein that specifically binds to PD-L1 comprises the following amino acid sequences: a heavy chain variable domain having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 7 or SEQ ID NO: 21; and a light chain variable domain having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 8 or SEQ ID NO: 22.

In some embodiments, the antigen binding protein that specifically binds to PD-L1 comprises the following amino acid sequences: a heavy chain variable domain as set forth in SEQ ID NO: 7; and a light chain variable domain as set forth in SEQ ID NO: 8.

In some embodiments, the antigen binding protein that specifically binds to PD-L1 comprises the following amino acid sequences: a heavy chain variable domain as set forth in SEQ ID NO: 21; and a light chain variable domain as set forth in SEQ ID NO: 22.

Preferably, the antigen binding protein that specifically binds to PD-L1 comprises the following amino acid sequences: a heavy chain amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 9 or SEQ ID NO: 23, or a heavy chain amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 29 or SEQ ID NO: 32; and a light chain amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 10 or SEQ ID NO: 24.

Preferably, the antigen binding protein that specifically binds to PD-L1 comprises the following amino acid sequences: a heavy chain amino acid sequence as set forth in SEQ ID NO: 9; and a light chain amino acid sequence as set forth in SEQ ID NO: 10.

Preferably, the antigen binding protein that specifically binds to PD-L1 comprises the following amino acid sequences: a heavy chain amino acid sequence as set forth in SEQ ID NO: 23; and a light chain amino acid sequence as set forth in SEQ ID NO: 24.

Preferably, the antigen binding protein that specifically binds to PD-L1 comprises the following amino acid sequences: a heavy chain amino acid sequence as set forth in SEQ ID NO: 29; and a light chain amino acid sequence as set forth in SEQ ID NO: 10.

Preferably, the antigen binding protein that specifically binds to PD-L1 comprises the following amino acid sequences: a heavy chain amino acid sequence as set forth in SEQ ID NO: 32; and a light chain amino acid sequence as set forth in SEQ ID NO: 24.

In certain embodiments, the fusion protein comprises: (1) two identical first polypeptides with an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 11 or SEQ ID NO: 25, or with an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 30 or SEQ ID NO: 33; and (2) two identical second polypeptides with an amino acid sequence having at least 80%, 81%, 82%, 83%, 84%, 85%, 86%, 87%, 88%, 89%, 90%, 91%, 92%, 93%, 94%, 95%, 96%, 97%, 98%, 99% or 100% identity to an amino acid sequence set forth in SEQ ID NO: 13 or SEQ ID NO: 27. Wherein, the amino acid sequence of the first polypeptide sequentially comprises, from the N terminus to the C terminus, the following sequences: a heavy chain variable domain of an antibody that recognizes PD-L1 antigenic epitope or antigen, an antibody constant region, a linker peptide, and a TGF-β binding fragment of a human TGF-β RII.

In another aspect, the present application further provides a polynucleotide sequence encoding the fusion protein described above. For example, the polynucleotide sequence comprises: a nucleic acid sequence of SEQ ID NO: 12 encoding an amino acid sequence set forth in SEQ ID NO: 11, a nucleic acid sequence of SEQ ID NO: 26 encoding an amino acid sequence set forth in SEQ ID NO: 25, a nucleic acid sequence of SEQ ID NO: 31 encoding an amino acid sequence set forth in SEQ ID NO: 30, or a nucleic acid sequence of SEQ ID NO: 34 encoding an amino acid sequence set forth in SEQ ID NO: 33; and a nucleic acid sequence of SEQ ID NO: 14 encoding an amino acid sequence set forth in SEQ ID NO: 13, or a nucleic acid sequence of SEQ ID NO: 28 encoding an amino acid sequence set forth in SEQ ID NO:27. Preferably, the polynucleotide sequence comprises nucleic acid sequences set forth in SEQ ID NO: 34 and SEQ ID NO: 28, or nucleic acid sequences set forth in SEQ ID NO: 31 and SEQ ID NO: 14. The present application further provides an expression vector comprising the polynucleotide sequence. Alternatively, the present application further provides a cell comprising the polynucleotide sequence or the expression vector.

In some aspects, the present application further relates to a pharmaceutical composition comprising the fusion protein.

In yet another aspect, the present application further provides a method for producing the fusion protein. The fusion protein comprises: (a) at least one heavy chain variable domain and at least one light chain variable domain of an antibody binding to human programmed death-ligand 1 (PD-L1); and (b) a human TGF-β RII or a TGF-β binding fragment or a variant thereof. The method comprises: preparing a recombinant DNA by gene recombination technology, introducing the recombinant DNA into a cell, and allowing the cell to stably express the protein. The cell can be selected from the group consisting of a bacterium, a yeast and a mammalian cell, and preferably a mammalian cell, e.g., CHO, NSO, COS and BHK cells. The method further comprises harvesting a culture of the cell and purifying the obtained protein.

The present application further provides use of the fusion protein or protein molecule in preparing drugs for treating cancer, inhibiting tumor growth or enhancing anti-tumor response. The treatment is selected based on factors such as the sensitivity of the subject to the therapy targeting PD-L1 and TGF-β, clinical experience, and the expression levels of PD-L1 and TGF-β in the subject. The present application further relates to a fusion protein for use in treating cancer, inhibiting tumor growth, or enhancing anti-tumor response.

In some aspects, the protein molecule or fusion protein described herein may be used for local depletion of TGF-β at a tumor site, and/or for blocking the signaling pathway of TGF-β in a cell (e.g., a tumor cell or an immune cell).

In some aspects, the protein molecule or the fusion protein described herein can be used for blocking the PD-L1 pathway, and/or promoting the killing of tumor cells by immune cells.

In some aspects, the protein molecule or the fusion protein described herein has use in treating cancer, inhibiting tumor growth, or enhancing anti-tumor response. The cancer or tumor includes, but is not limited to, lung adenocarcinoma, mucinous adenocarcinoma, low-grade brain neuroglioma, glioblastoma multiforme, mesothelioma, melanoma, thyroid cancer, renal cancer, liver cancer, acute myeloid leukemia, esophageal adenocarcinoma, lymphoma, non-small cell lung cancer, metastatic non-small cell lung cancer, advanced or recurrent non-small cell lung cancer, refractory non-small cell lung cancer after chemotherapy, metastatic non-squamous non-small cell lung cancer, advanced or recurrent non-squamous non-small cell lung cancer, unresectable advanced non-small cell lung cancer, occult non-small cell lung cancer, breast cancer, metastatic breast cancer, triple-negative breast cancer, advanced or recurrent breast cancer, locally recurrent breast cancer, inflammatory breast cancer, pancreatic ductal adenocarcinoma, metastatic pancreatic cancer, locally advanced unresectable pancreatic cancer, recurrent pancreatic cancer, prostate cancer, advanced or metastatic prostate cancer, locally advanced prostate cancer, castration-resistant prostate cancer, recurrent prostate cancer after castration, localized prostate cancer, progressive prostate cancer, colorectal cancer, rectal adenocarcinoma, large intestinal cancer, metastatic colorectal cancer, advanced or recurrent colon cancer, advanced or recurrent rectal cancer, locally recurrent rectal cancer, locally recurrent colon cancer, gastric adenocarcinoma, gastric cancer, unresectable gastric cancer, metastatic gastric cancer, locally advanced or recurrent gastric cancer, gastrointestinal stromal tumor, biliary tract cancer, bile duct cancer, gallbladder cancer, unresectable or metastatic biliary tract cancer, unresectable or metastatic bile duct cancer, unresectable or metastatic gallbladder cancer, penile cancer, anal cancer, vaginal cancer, cervical cancer, locally advanced cervical cancer, recurrent cervical cancer, metastatic cervical cancer, metastatic penile cancer, advanced or recurrent vaginal squamous cell carcinoma, advanced or recurrent vaginal adenocarcinoma, uterine corpus endometrioid carcinoma, bladder cancer, human papillomavirus infection, head and neck cancer, recurrent or metastatic head and neck cancer, hypopharyngeal cancer, laryngeal cancer, oral cancer, nasopharyngeal carcinoma, oropharyngeal cancer, pharyngolaryngeal cancer, paranasal sinus and nasal cavity cancer, and salivary gland cancer. The use may be to administer the protein molecule or the fusion protein, or a pharmaceutical composition comprising the protein molecule or the fusion protein, alone or in combination with other tumor therapies, such as radiotherapy, chemotherapy, surgery, biologics, and chemicals.

The present application further provides a method for treating cancer, inhibiting tumor growth, or enhancing anti-tumor response. The method comprises administering the protein molecule or the fusion protein, or a pharmaceutical composition comprising the protein molecule or the fusion protein, alone or in combination with other tumor therapies, such as radiotherapy, chemotherapy, surgery, biologics, and chemicals. The tumor or cancer includes, but is not limited to, lung adenocarcinoma, mucinous adenocarcinoma, low-grade brain neuroglioma, glioblastoma multiforme, mesothelioma, melanoma, thyroid cancer, renal cancer, liver cancer, acute myeloid leukemia, esophageal adenocarcinoma, lymphoma, non-small cell lung cancer, metastatic non-small cell lung cancer, advanced or recurrent non-small cell lung cancer, refractory non-small cell lung cancer after chemotherapy, metastatic non-squamous non-small cell lung cancer, advanced or recurrent non-squamous non-small cell lung cancer, unresectable advanced non-small cell lung cancer, occult non-small cell lung cancer, breast cancer, metastatic breast cancer, triple-negative breast cancer, advanced or recurrent breast cancer, locally recurrent breast cancer, inflammatory breast cancer, pancreatic ductal adenocarcinoma, metastatic pancreatic cancer, locally advanced unresectable pancreatic cancer, recurrent pancreatic cancer, prostate cancer, advanced or metastatic prostate cancer, locally advanced prostate cancer, castration-resistant prostate cancer, recurrent prostate cancer after castration, localized prostate cancer, progressive prostate cancer, colorectal cancer, rectal adenocarcinoma, large intestinal cancer, metastatic colorectal cancer, advanced or recurrent colon cancer, advanced or recurrent rectal cancer, locally recurrent rectal cancer, locally recurrent colon cancer, gastric adenocarcinoma, gastric cancer, unresectable gastric cancer, metastatic gastric cancer, locally advanced or recurrent gastric cancer, gastrointestinal stromal tumor, biliary tract cancer, bile duct cancer, gallbladder cancer, unresectable or metastatic biliary tract cancer, unresectable or metastatic bile duct cancer, unresectable or metastatic gallbladder cancer, penile cancer, anal cancer, vaginal cancer, cervical cancer, locally advanced cervical cancer, recurrent cervical cancer, metastatic cervical cancer, metastatic penile cancer, advanced or recurrent vaginal squamous cell carcinoma, advanced or recurrent vaginal adenocarcinoma, uterine corpus endometrioid carcinoma, bladder cancer, human papillomavirus infection, head and neck cancer, recurrent or metastatic head and neck cancer, hypopharyngeal cancer, laryngeal cancer, oral cancer, nasopharyngeal carcinoma, oropharyngeal cancer, pharyngolaryngeal cancer, paranasal sinus and nasal cavity cancer, and salivary gland cancer.

## Definitions

Unless otherwise specified, technical and scientific terms used herein have the same meaning as generally understood by those of ordinary skills in the art. If there are multiple definitions for a term in the present application, the definition used in this section shall be adopted unless otherwise specified.

The term "protein molecule" is sometimes referred to as the fusion protein herein.

The term "TGF-β RII" or "TGF-β receptor II" refers to a polypeptide or protein having a wild-type human TGF-β receptor type 2 isotype B sequence, e.g., a polypeptide set forth in SEQ ID NO: 35, or a polypeptide having a sequence substantially identical to an amino acid sequence set forth in SEQ ID NO: 35.

The term "fragment that binds to TGF-β" or "TGF-β binding fragment" of TGF-β RII refers to a fragment of TGF-β RII that has TGF-β binding activity and accounts for about at least 0.1%, 0.5%, 1%, 5%, 10%, 25%, 35%, 50%, 75%, 90%, 95%, 99% or 100% of the TGF-β RII sequence. The fragment is generally a soluble fragment, e.g., an extracellular domain of a human TGF-β RII or a variant thereof.

As used herein, the term "antibody" refers to a binding protein having at least one antigen binding domain. The antibody and the fragment thereof of the present application can be an intact antibody or any fragment thereof. Thus, the antibody and the fragment thereof of the present application include a monoclonal antibody or a fragment thereof and an antibody variant or a fragment thereof, as well as an immunoconjugate. Examples of the antibody fragment include a Fab fragment, a Fab' fragment, a $F(ab)'_2$ fragments, a Fv fragment, a Fd' fragment, an isolated CDR, a single-chain Fv molecule (scFv), and other antibody fragments known in the art, as well as antibodies with any modification known in the art (e.g., glycosylation, chemical modification, and the like). The antibody and the fragment thereof may also include a recombinant polypeptide, a fusion protein, and a bispecific antibody. The anti-PD-L1 antibody and the fragment thereof disclosed herein can be of IgG1, IgG2, IgG3, or IgG4 isotype. The term "isotype" refers to the class of antibodies encoded by the heavy chain constant region gene. The antibody and the fragment thereof may be a chimeric antibody, a humanized antibody or an intact human antibody.

The term "chimeric antibody" refers to an antibody having at least a portion of a heavy chain variable domain and a portion of a light chain variable domain derived from one species, and at least a portion of a constant region derived from another species. For example, in one embodiment, the chimeric antibody may comprise murine variable domains and a human constant region.

The term "humanized antibody" refers to an antibody comprising complementarity determining regions (CDRs) derived from a non-human antibody, and framework and constant regions derived from a human antibody. For example, the anti-PD-L1 antibody may comprise CDRs derived from one or more murine antibodies and human framework and constant regions. Exemplary humanized antibodies are disclosed herein. Additional anti-PD-L1 antibodies or variants thereof comprising the heavy and light chain CDRs disclosed herein can be generated using any human framework sequences, and are also included in the present application. In one embodiment, framework sequences suitable for use in the present application include those similar in structure to the framework sequences disclosed herein. Additional modifications may be made in the framework regions to improve the properties of the antibodies disclosed herein. Such additional framework modifications may include: chemical modifications, point mutations for reducing immunogenicity or removing T cell epitopes, or modifications reverting the mutations to residues in original germline sequences. In some embodiments, such modifications include those corresponding to the mutations exemplified herein, including reversions to germline sequences. For example, in one embodiment, one or more amino acids in the human VH and/or VL framework regions of the humanized antibodies disclosed herein are reverted to the corresponding amino acids in the parent murine antibodies.

As used herein, the term "derived", when used to refer to a molecule or polypeptide relative to a reference antibody or other binding proteins, means a molecule or polypeptide that can specifically bind to the same epitope as the reference antibody or other binding proteins.

As used herein, the term "$EC_{50}$" refers to the effective concentration, 50% of the maximal response of an antibody. As used herein, the term "$IC_{50}$" refers to the inhibitory concentration, 50% of the maximal response of an antibody. Both $EC_{50}$ and $IC_{50}$ can be measured by ELISA or FACS assay or any other method known in the art.

The term "antigen binding fragment" refers to a fragment that retains the antigen binding function of a full-length antibody, including Fab, Fab', $F(ab')_2$, scFv, Fv, Fd, Fd', an isolated CDR, a single-domain VHH fragment, and other antibody fragments known in the art, or fragments obtained by making any modification known in the art to the above fragments.

The term "linker peptide" refers to a polypeptide or peptide segment, preferably having synthetic amino acid sequences, that links the C terminus of an antibody or an antigen binding fragment thereof to the N terminus of the TGF-β binding domain in the fusion protein. The linker peptide used herein may be selected from $(G_4S)_xG$, wherein x is optionally an integer of 3-6, preferably 4-5, and most preferably 4.

The term "identity" refers to the similarity between two reference sequences. The percent identity refers to a percentage that describes how similar sequences or designated regions of the sequences are by comparison using a sequence comparison algorithm known to those skilled in the art.

The term "substantially identical" refers to that the sequences have at least about 80% and higher (e.g., 81%, 82%, 83%, 84%, 85%, 86%, 87%, 88%, 89%, 90%, 91%, 92%, 93%, 94%, 95%, 96%, 97%, 98%, 99%, or 100%) identity.

The term "subject" refers to a mammal, including a human and a non-human animal, and preferably a human.

The term "treating" includes therapeutic treatment and prophylactic treatment or preventative measures, in which a therapeutic agent is administered to the subject to reduce at least one symptom of a disease, disorder, or condition (e.g., cancer or tumor), or to relieve the symptoms.

The term "cancer" refers to a collection of cells that proliferate in an abnormal manner.

Unless otherwise specified, terms in the singular shall be deemed to include the plural and vice versa. Unless otherwise specified, the word "a" or "an" refers to "at least one". Unless otherwise stated, use of "or" means "and/or".

Unless otherwise specified, the term "about" described herein refers to a fluctuation within ±5%, preferably within ±2%, and more preferably within ±1%, of the specified numerical range given.

As described herein, any percentage range, ratio range, or integer range shall be understood as including the value of any integer within the listed range and including, when appropriate, fractions thereof (such as one tenth and one hundredth of the integer) unless otherwise indicated.

As used herein, the terms "comprise", "comprises" and "comprising" or equivalents thereof are open-ended statements and mean that elements, components and steps that are not specified may be included in addition to those listed, unless otherwise stated.

All patents, patent applications and other identified publications are expressly incorporated herein by reference for the purpose of description and disclosure. These publications are provided solely because they were disclosed prior to the filing date of the present application. All statements as to the dates of these documents or description as to the contents of these documents are based on the information available to the applicant and do not constitute any admission as to the correctness of the dates or the content of these documents. Moreover, in any country or region, any reference to these publications herein is not to be construed as an admission that the publications form part of the commonly recognized knowledge in the art.

## BRIEF DESCRIPTION OF THE DRAWINGS

FIG. 1 is a structural schematic of an anti-PD-L1-TGF-β RII fusion protein, in which the C terminus of the heavy chain of the anti-PD-L1 antibody is linked to the N terminus of the TGF-β receptor II extracellular domain by a $(G_4S)_4G$ linker peptide.

FIG. 2A shows the biological activity of the PD-L1 terminus of hu5G11-hIgG1-TGF-β RII detected by reporter gene assay; FIG. 2B shows the biological activity of the PD-L1 terminus of M7824 detected by reporter gene assay .

FIG. 3 shows the *in vitro* binding activity of hu5G11-hIgG1-TGF-β RII to PD-L1 detected by ELISA assay.

FIG. 4 shows the *in vitro* binding activity of hu5G11-hIgG1-TGF-β RII to TGF-β1 detected by ELISA assay.

## EXAMPLES

The present application is further described below with reference to specific examples, which, however, are only for illustration and do not limit the scope of the present application. Likewise, the present application is not limited to any specific preferred embodiment described herein. It should be appreciated by those skilled in the art that equivalent substitutions or corresponding modifications made to the technical features of the present application still fall within the scope of the present application. Unless otherwise stated, the reagents used in the following examples are commercially available products, and the solutions can be prepared by conventional techniques in the art.

Example 1 Construction and Expression of Fusion Protein Transient Expression Plasmid

An anti-PD-L1-TGF-β RII fusion protein hu5G11-hIgG1-TGF-β RII was constructed, as shown in FIG. 1. The light chain of this molecule had an amino acid sequence of SEQ ID NO: 27. The heavy chain (SEQ ID NO: 33) of this molecule was a fusion protein comprising a heavy chain of an anti-human-PD-L1 antibody (SEQ ID NO: 32), an amino acid sequence (SEQ ID NO: 36) of a human TGF-β RII extracellular domain, and a linker peptide $(G_4S)_4G$ (SEQ ID NO: 37) linking the former two.

The light chain amino acid sequence (SEQ ID NO: 27) of the hu5G11-hIgG1-TGF-β RII was:

DIQMTQSPSSLSASVGDRVTITCKASQSVSNDVAWYQQKPGKAPKLLIYYAANRYTGVPDRFSGSGYGTD
FTFTISSLQPEDIATYFCQQDYTSPYTFGQGTKLEIKRTVAAPSVFIFPPSDEQLKSGTASVVCLLNNFYPREA
KVQWKVDNALQSGNSQESVTEQDSKDSTYSLSSTLTLSKADYEKHKVYACEVTHQGLSSPVTKSFNRGEC

The heavy chain amino acid sequence (SEQ ID NO: 33) of the hu5G11-hIgG1-TGF-β RII was:

QITLKESGPTLVKPTQTLTLTCTVSGFSLSTYGVHWIRQPPGKALEWLGVIWRGVTTDYNAAFMSRLTIT
KDNSKNQVVLTMNNMDPVDTATYYCARLGFYAMDYWGQGTLVTVSSASTKGPSVFPLAPSSKSTSGG
TAALGCLVKDYFPEPVTVSWNSGALTSGVHTFPAVLQSSGLYSLSSVVTVPSSSLGTQTYICNVNHKPSN
TKVDKKVEPKSCDKTHTCPPCPAPELLGGPSVFLFPPKPKDTLMISRTPEVTCVVVAVSHEDPEVKFNWY
VDGVEVHNAKTKPREEQYNSTYRVVSVLTVLHQDWLNGKEYKCKVSNKALPAPIEKTISKAKGQPREP
QVYTLPPSRDELTKNQVSLTCLVKGFYPSDIAVEWESNGQPENNYKTTPPVLDSDGSFFLYSKLTVDKSR
WQQGNVFSCSVMHEALHNHYTQKSLSLSPGAGGGGSGGGGSGGGGSGGGGSGIPPHVQKSVNNDMIVT
DNNGAVKFPQLCKFCDVRFSTCDNQKSCMSNCSITSICEKPQEVCVAVWRKNDENITLETVCHDPKLPY
HDFILEDAASPKCIMKEKKKPGETFFMCSCSSDECNDNIIFSEEYNTSNPD

The heavy chain amino acid sequence (SEQ ID NO: 32) of the PD-L1 antibody was:

QITLKESGPTLVKPTQTLTLTCTVSGFSLSTYGVHWIRQPPGKALEWLGVIWRGVTTDYNAAFMSRLTIT KDNSKNQVVLTMNNMDPVDTATYYCARLGFYAMDYWGQGTLVTVSSASTKGPSVFPLAPSSKSTSGG TAALGCLVKDYFPEPVTVSWNSGALTSGVHTFPAVLQSSGLYSLSSVVTVPSSSLGTQTYICNVNHKPSN TKVDKKVEPKSCDKTHTCPPCPAPELLGGPSVFLFPPKPKDTLMISRTPEVTCVVVAVSHEDPEVKFNWY VDGVEVHNAKTKPREEQYNSTYRVVSVLTVLHQDWLNGKEYKCKVSNKALPAPIEKTISKAKGQPREP QVYTLPPSRDELTKNQVSLTCLVKGFYPSDIAVEWESNGQPENNYKTTPPVLDSDGSFFLYSKLTVDKSR WQQGNVFSCSVMHEALHNHYTQKSLSLSPGA

The human TGF-β RII extracellular domain amino acid sequence (SEQ ID NO: 36) was:

IPPHVQKSVNNDMIVTDNNGAVKFPQLCKFCDVRFSTCDNQKSCMSNCSITSICEKPQEVCVAVWRKND ENITLETVCHDPKLPYHDFILEDAASPKCIMKEKKKPGETFFMCSCSSDECNDNIIFSEEYNTSNPD

The amino acid sequence (SEQ ID NO: 37) of the linker peptide was: GGGGSGGGGSGGGGSGGGGSG

A whole gene sequence containing the heavy chain nucleic acid sequence (SEQ ID NO: 34) and the light chain nucleic acid sequence (SEQ ID NO: 28) of the anti-PD-L1y-TGF-β RII fusion protein was synthesized and then incorporated to a eukaryotic expression vector, preferably a pcDNA3.1(+) expression vector, to construct the target expression plasmid. The plasmid was extracted to prepare the target plasmid. The expression plasmids for heavy and light chains were introduced into ExpiCHO-S™ cells using the ExpiFectamine™ CHO transfection technique for transient expression of the target protein. The obtained cell culture supernatant was purified to give the protein.

Example 2 Isolation, Purification and Identification of Fusion Protein

The cell culture supernatant obtained in Example 1 was loaded onto a protein A affinity chromatography column, such as MabSelect from the GE company. The chromatography column was equilibrated with a phosphate equilibration buffer (e.g., 10 mM phosphate buffer, pH = 6.0), and rinsed with a rinse buffer containing sodium chloride (e.g., a 25 mM phosphate buffer containing 0.5 M sodium chloride, pH = 7.0-7.4) to remove partially bound impurities. Finally, the target protein product bound to the chromatography column were eluted with an eluent buffer to provide the anti-PD-L1-TGF-β RII fusion protein (i.e., hu5G11-hIgG1-TGF-β RII). The elution can be carried out by conventional methods, for example, using high-salt buffer or changing pH, such as using 1 M arginine hydrochloride buffer (pH = 3-4) or 50 mM citrate buffer (pH = 3-4) for elution. The phosphate buffer was prepared from disodium hydrogen phosphate and sodium dihydrogen phosphate, and the citrate buffer was prepared from citric acid and trisodium citrate. The eluate was quantified by UV analysis.

Example 3 *In-Vitro* Binding Affinity Assay of Fusion Protein

By using Biacore T200, the affinities of the fusion protein hu5G11-hIgG1-TGF-β RII and M7824 to PD-L1 and TGF-β were analyzed. M7824 is the anti-PD-L1/TGF-β trap in Patent No. CN106103488, and the heavy and light chain sequences are set forth in SEQ ID NOs: 42 and 43 of the present application, respectively. The DNA sequences encoding heavy and light chains were cloned into expression vectors after synthesis. The light and heavy chain expression vectors were co-transfected into ExpiCHO-S™. The cells were incubated in an incubator at 37 °C with 8% $CO_2$. The culture supernatant was purified using a protein A filler as described in Example 2 to give M7824.

1) Affinity assay of the fusion protein to PD-L1: An anti-hIgG1(Fc) antibody was immobilized on the surface of a CM5 chip by amino coupling in an amount of about 8000-9000 RU. The fusion protein hu5G11-hIgG1-TGF-β RII and M7824 were captured using the CM5 chip. After diluting the PD-L1 protein (Sinobiological, 10084-H08H) using a running buffer to 20 nM, 10 nM, 5 nM, 2.5 nM and 1.25 nM, signals of interactions of the different concentrations of PD-L1 protein and the blank control (the running buffer) with the fusion protein hu5G11-hIgG1-TGF-β RII and M7824 were assayed, so as to obtain association and dissociation curves, The chip was regenerated with 3 mol/L $MgCl_2$ buffer to the baseline.

2) Affinity assay of the fusion protein to TGF-β: An anti-hIgG1(Fc) antibody was immobilized on the surface of a CM5 chip by amino coupling in an amount of about 8000-9000 RU. The fusion protein hu5G11-hIgG1-TGF-β RII and M7824 were captured using the CM5 chip. After diluting the TGF-β protein (Sinobiological, 10804-H08H) using a running buffer to 1000 nM, 500 nM, 250 nM, 125 nM and 62.5 nM, signals of interactions of the different concentrations of TGF-β protein and the blank control (the running buffer) with the fusion protein hu5G11-hIgG1-TGF-β RII and M7824 were assayed, so as to obtain association and dissociation curves, The chip was regenerated with 3 mol/L $MgCl_2$ buffer to the baseline.

The data signals were collected by BiaControl Software 2.0 in real time, and analyzed by BiaEvaluation Software 2.0. The data were fitted using a Langmuir 1:1 model after being subtracted (i.e., blank control signals were subtracted from sample signals in each cycle), so as to calculate the association rate constant Ka, the dissociation rate constant Kd and the equilibrium constant KD.

Table 1. Affinity of test samples to PD-L1 and TGF-β

| PD-L1 terminus | | | |
|---|---|---|---|
| Sample | KD (M) | ka(1/Ms) | kd(1/s) |
| hu5G11-hIgG1-TGFβ RII | 4.27E-11 | 1.74E+07 | 7.45E-04 |
| M7824 | 7.37E-11 | 5.15E+07 | 3.79E-03 |
| TGF-β terminus | | | |
| Sample | KD (M) | ka(1/Ms) | kd(1/s) |
| hu5G11-hIgG1-TGFβ RII | 1.13 E-08 | 8.28 E+04 | 9.37E-04 |
| M7824 | 1.52 E-08 | 2.44E+05 | 3.71E-03 |

As can be seen from the above table, the fusion protein of the present application, as compared to the reference M7824, has smaller KD and Kd values, i.e., the fusion protein of the present application has higher affinity to both PD-L1 and TGF-β.

Example 4 Biological activity assay of PD-L1 terminus of hu5G11-hIgG1-TGF-β RII by reporter gene assay

CHO-PDL1-CD3L cells (National Institutes for Food and Drug Control) were taken, adjusted to a viable cell density of 4-5 $\times 10^5$ cells/mL using a CHO-PDL1-CD3L complete medium, added to a white 96-well cell plate at 100 μL/well, and incubated in a cell incubator at 37 °C with 5% $CO_2$ for 16-20 h. Jurkat-PD-1-NFAT cells (National Institutes for Food and Drug Control) in the logarithmic growth phase were taken, and adjusted to a viable cell density of 1.25-2 $\times 10^6$ cells/mL using an assay medium. The cell plate was taken out, and the supernatant was discarded. The Jurkat-PD-1-NFAT cell suspension was added to the above cell plate at 50 μL/well. hu5G11-hIgG1-TGF-β RII was pre-diluted to a concentration of 56 nmol/L and then serially 2-fold diluted to the 10$^{th}$ concentration (i.e., about 56 nmol/L, about 28 nmol/L, about 14 nmol/L, about 7 nmol/L, about 3.5 nmol/L, about 1.75 nmol/L, about 0.875 nmol/L, about 0.4375 nmol/L, about 0.21875 nmol/L, and about 0.109375 nmol/L). The PD-L1 antibody was pre-diluted to a concentration of 68 nmol/L and then serially 2-fold diluted to the 10$^{th}$ concentration. M7824 was pre-diluted to a concentration of 56 nmol/L and then serially 2-fold diluted to the 10$^{th}$ concentration. The serially diluted hu5G11-hIgG1-TGF-β RII, PD-L1 antibody and M7824 were added to the cell plate at 50 μL/well, and incubated in a cell incubator with 5% $CO_2$ at 37 °C for 6 h. Bio-Glo Luciferase reagent (Promega, G7940) was added to the above cell plate at 100 μL/well, and the cells were incubated in the dark at room temperature for 2-3 min. Relative light unit (RLU) was read by a multifunctional microplate reader (Thermo, Varioskan Flash). The biological activity results of the PD-L1 terminus of the fusion protein hu5G11-hIgG1-TGF-β RII described in the examples of the present application are shown in FIG. 2A and Table 2. The results in Table 2 indicate that the PD-L1 terminus of the fusion protein of the present application has higher biological activity.

$$\text{Biological activity (\%) of test sample} = (\text{EC}_{50} \text{ value of reference sample} / \text{EC}_{50} \text{ value of test sample}) \times 100\%$$

Table 2. Biological activity of PD-L1 terminus of test samples measured by reporter gene assay.

| Sample | Biological activity | $EC_{50}$ (nmol/L) |
|---|---|---|
| PD-L1 antibody | 100% | 4.603 |
| hu5G11-hIgG1-TGFβ RII | 75.4% | 6.106 |

(continued)

| Sample | Biological activity | $EC_{50}$ (nmol/L) |
|---|---|---|
| PD-L1 antibody | 100% | 4.151 |
| M7824 | 72.5% | 5.724 |

Note: The PD-L1 antibody (the reference sample) was a humanized 5G11-IgG1 antibody described in Patent No, CN107001463 of heavy and light chain sequences set forth in SEQ ID NOs: 23 and 24 of the present application respectively. M7824 was an anti-PD-L1/TGF-β trap described in Patent No, CN106103488 with heavy and light chain sequences set forth in SEQ ID NOs: 42 and 43, respectively. See Example 3 for the preparation method.

Example 5 *In-vitro* Binding Activity Assay of Fusion Protein to PD-L1 and TGF-β by Enzyme-linked Immunosorbent Assay (ELISA).

*In-vitro* binding assay of fusion protein to PD-L1

1) Coating: A PD-L1 recombinant protein (Sinobiological, 10084-H08H) was diluted with PBS to 2 (μg/mL, immobilized on the 96-well plate at 100 μL/well, and incubated overnight at 4 °C.

2) The plate was washed once with PBS + 0.05% Tween 20, and a blocking buffer (PBS + 3% BSA solution) was added at 250 μL/well for 1-2 h of blocking.

3) hu5G11-hIgG1-TGF-β RII was pre-diluted with a diluent (PBS + 0.05% Tween 20 + 1% BSA) to about 4000 ng/mL, and then serially 4-fold diluted to the $7^{th}$ concentration (i.e., about 4000 ng/mL, about 1000 ng/mL, about 250 ng/mL, about 62.5 ng/mL, about 15.625 ng/mL, about 3.90625 ng/mL and about 0.9765625 ng/mL). After the PD-L1 recombinant protein was washed 3 times with PBS + 0.05% Tween 20, the serially diluted fusion protein hu5G11-hIgG1-TGF-β RII and blank control were added at 100 μL/well, and incubated at 25 °C for 1-2 h.

4) The plate was washed 3 times with PBS + 0.05% Tween 20, and an HRP-labeled goat anti-human secondary antibody (PE, NEF802001EA) diluted in a 1:3500 ratio was added at 100 μL/well, The plate was incubated at 25 °C for 1 h.

5) The plate was washed 3 times with PBS + 0.05% Tween 20 before 3,3',5,5'-tetramethylbenzidine (TMB) was added at 100 μL/well, The plate was incubated at 25 °C for 5 min in the dark. Finally, 1 M $H_2SO_4$ was added to terminate the reaction.

6) The absorbance was measured at 450 nm with a microplate reader (Thermo Scientific, Varioskan Flash). A curve was plotted with the average absorbance as the abscissa and the logarithmic concertration of hu5G11-hIgG1-TGFβ RII as the ordinate. Then the $EC_{50}$ of hu5G11-hIgG1-TGF-β RII was calculated.

*In-vitro* binding assay of fusion protein to TGF-β

1) Coating: A TGF-β1 recombinant protein (Sinobiological, 10804-H08H) was diluted with PBS to 2 μg/mL, immobilized on the 96-well plate at 100 μL/well, and incubated overnight at 4 °C; the other procedures are as described above.

The results of the *in-vitro* binding assay of the fusion protein hu5G11-hIgG1-TGF-β RII in the examples of the present application to PD-L1 are shown in FIG. 3 and Table 3, while the results of the *in-vitro* binding assay of the fusion protein to TGF-β1 are shown in FIG. 4 and Table 3. The ELISA results demonstrate that the fusion protein retains the binding activity to PD-L1 and TGF-β.

Table 3. $EC_{50}$ of hu5G11-hIgG1-TGF-β RII binding to PD-L1 and TGF-β by ELISA

| | PD-L1 terminus | TGF-β terminus |
|---|---|---|
| Sample | $EC_{50}$ (ng/ml) | $EC_{50}$ (ng/ml) |
| PD-L1/TGFβ RII | 34.31 | 67.03 |

Example 6 Pharmacokinetic Analysis

1) Single-dose pharmacokinetic study: Cynomolgus monkeys as animal model were randomly divided into 3 groups of 6, half male and half female. The groups were infused intravenously with a single dose of 1, 10 and 60 mg/kg of fusion protein hu5G11-hIgG1-TGF-β RII. Whole blood samples were collected from the veins at 0 h pre-dose and at 1 min, 3 h, 8 h, 24 h, 48 h, 72 h, 120 h, 168 h, 216 h, 264 h, 336 h, 504 h and 672 h post-dose. The supernatant was separated, and parameters such as blood concentration were detected by ELISA. See Table 4 for the results.

2) Repeat-dose pharmacokinetic study: Cynomolgus monkeys as animal model, were randomly divided into 3 groups of 10, half male and half female. The monkeys were infused intravenously with 20, 60 and 200 mg/kg of fusion protein hu5G11-hIgG1-TGF-β RII once weekly for 4 weeks (5 doses in total). The blood was collected from the veins at 0 h before the first dose and at 1 min, 3 h, 8 h, 24 h, 48 h, 72 h, 120 h, and 168 h after the start of the first dose, at 0 h before the third and fourth doses and at 1 min after the start of the third and fourth doses, and at 0 h before the fifth dose and at 1 min, 3 h, 8 h, 24 h, 48 h, 72 h, 120 h, 168 h, 216 h, 264 h, 336 h, 504 h and 672 h after the start of the fifth dose. The supernatant was separated, and parameters such as blood concentration in the supernatant were detected by ELISA. See Table 5 for the results. The results in Table 5 indicate that no significant accumulation in the serum for hu5G11-hIgG1-TGF-β RII was observed.

The ELISA procedures are as follows:
1) Coating: A human PD-L1/B7-H1/CD274 Protein, Fc ta (Sinobiological, LC11NO2402) was diluted with PBS to 1 μg/mL, immobilized on a 96-well plate at 100 μL/well, and incubated overnight at 4 °C;
2) The plate was washed 3 times with PBS + 0.05% Tween 20, and a blocking buffer (a solution of PBS + 0.05% Tween 20 + 1% BSA) was added at 300 μL/well at 25 °C for 2-3 h of blocking;
3) The plate was washed 3 times with PBS + 0.05% Tween 20, and blank control samples, standard curve samples and test samples were added at 100 μL/well. The plate was incubated at 25 °C for 1-1.5 h;
4) The plate was washed 3 times with PBS + 0.05% Tween 20. A human TGF-β RII biotinylated antibody (R&D, XL0519051) was diluted with a blocking buffer to a final concentration of 0.05 μg/mL and added to the plate at 100 μL/well. The plate was incubated at 25 °C for 1-1.5 h;
5) The plate was washed 3 times with PBS + 0.05% Tween 20, and streptavidin-HRP diluted 1:200 with a blocking buffer was added at 100 μL/well. The plate was incubated at 25 °C for 30 min;
6) The plate was washed 3 times with PBS + 0.05% Tween 20, and a TMB substrate was added at 100 μL/well. The plate was incubated in the dark at 25 °C for 5-10 min. Finally, 0.5 M $H_2SO_4$ was added at 100 μL/well to terminate the reaction.
7) The absorbance value at 450 nm was measured using a microplate reader.

Table 4. Single-dose pharmacokinetics for hu5G11-hIgG1-TGF-β RII

| Parameters | Unit | Single dose | | | | | | | | |
|---|---|---|---|---|---|---|---|---|---|---|
| | | 1 mg/kg | | | 10 mg/kg | | | 60 mg/kg | | |
| | | Female | Male | Mean | Female | Male | Mean | Female | Male | Mean |
| $AUC_{INF_obs}$ | h*μg/mL | 444±164 | 460±162 | 452±146 | 8150±311 | 8270±558 | 8210±410 | 44500±10500 | 55200±10200 | 49800±10900 |
| $AUC_{last}$ | h*μg/mL | 434±170 | 447±165 | 441±150 | 8040±341 | 7980±551 | 8010±411 | 44200±11000 | 55000±10400 | 49600±11200 |
| $Cl_{_obs}$ | mL/h/kg | 2.47±0.924 | 2.42±1.04 | 2.45±0.879 | 1.23±0.0462 | 1.21±0.0850 | 1.22±0.0618 | 1.41±0.383 | 1.11±0.195 | 1.26±0.316 |
| $C_{max}$ | μg/mL | 20.6±1.51 | 19.2±0.819 | 19.9±1.33 | 214±16.0 | 220±13.5 | 217±13.7 | 1360±80.8 | 1260±72.3 | 1310±90.1 |
| $MRT_{INF_obs}$ | h | 35.2±12.0 | 43.1±10.2 | 39.2±10.8 | 66.7±3.50 | 81.2±8.44 | 74.0±9.85 | 84.0±11.6 | 109±9.98 | 96.6±16.9 |
| $MRT_{last}$ | h | 32.1±13.5 | 38.1±11.8 | 35.1±11.8 | 63.5±2.86 | 72.5±6.54 | 68.0±6.69 | 80.1±15.6 | 107±7.39 | 93.8±18.5 |
| $T_{1/2}$ | h | 26.5±7.55 | 33.2±5.97 | 29.8±7.11 | 42.0±7.06 | 36.7±31.1 | 39.3±20.4 | 56.1±34.4 | 66.6±38.9 | 61.3±33.4 |
| Tmax | h | 0.0167±0.00 | 0.0167±0.00 | 0.0167±0.00 | 0.0167±0.00 | 0.0167±0.00 | 0.0167±0.00 | 0.0167±0.00 | 0.0167±0.00 | 0.0167±0.00 |
| $V_{ss_obs}$ | mL/kg | 80.0±2.87 | 97.3±15.4 | 88.6±13.7 | 81.8±2.95 | 98.2±8.43 | 90.0±10.6 | 116±22.5 | 122±29.0 | 119±23.5 |

Table 5. Repeated-dose pharmacokinetics for hu5G11-hIgG1-TGF-β RII

| Dosage | Time | Gender | Cmax (mg /mL) | $AUC_{0\text{-}168h}$ (h*mg/mL) |
|---|---|---|---|---|
| 20 mg/kg | First dose | Female | 0.467±0.0911 | 15.9±3.97 |
| | | Male | 0.484±0.116 | 17.4±5.40 |
| | Last dose | Female | 0.282±0.222 | 4.74±6.53 |
| | | Male | 0.366±0.284 | 15.3±17.4 |
| 60 mg/kg | First dose | Female | 1.36±0.126 | 43.9±8.56 |
| | | Male | 1.43±0.212 | 52.8±13.0 |
| | Last dose | Female | 1.48±0.398 | 45.8±17.0 |
| | | Male | 1.25±0.459 | 35.9±19.7 |
| 200 mg/kg | First dose | Female | 5.96±0.640 | 176±31.2 |
| | | Male | 4.42±0.772 | 168±35.4 |
| | Last dose | Female | 5.50±1.32 | 209±86.7 |
| | | Male | 5.62±1.11 | 210±40.8 |

Example 7 Inhibitory Effect of Fusion Protein on Mouse MC38 Graft Tumor

The DNA sequence of IgG1 was synthesized and cloned into an expression vector pcDNA3.1. Light and heavy chain expression vectors were co-transfected into ExpiCHO-S™ using an ExpiCHO transfection kit (Thermo Fisher, A29133). The cells were cultured in an incubator with 8% $CO_2$ at 37 °C, and then the culture supernatant was purified using protein A filler as described in Example 2 to give IgG1. The heavy and light chain amino acid sequences of IgG1 are set forth in SEQ ID NO: 38 and SEQ ID NO: 39 of the present application.

The DNA sequence of IgG1-TGF-β RII was synthesized and cloned into an expression vector pcDNA3.1. Light and heavy chain expression vectors were co-transfected into ExpiCHO-STM using an ExpiCHO transfection kit (Thermo Fisher, A29133). The cells were cultured in an incubator with 8% $CO_2$ at 37 °C, and then the culture supernatant was purified using protein A filler as described in Example 2 to give IgG1-TGFβ RII. The heavy and light chain amino acid sequences of IgG1-TGF-β RII are set forth in SEQ ID NO: 40 and SEQ ID NO: 41 of the present application, respectively.

Humanized PD-L1 mice were subcutaneously inoculated with $3 \times 10^5$ MC38/hPD-L1 cells/mouse to establish mouse colon cancer models. When tumors grew to 50-70 $mm^3$, the mice were divided into groups of 10 according to the tumor volume, and then intravenously (IV) injected with 6 doses of hu5G11-hIgG1-TGF-β RII, IgG1 or IgG1-TGF-β RII once every two days in an injection volume of 0.1 mL/10 g body weight. The diameters of the tumors were measured twice weekly with a vernier caliper. The effect of the drug on tumor growth was examined based on the obtained T/C% or tumor growth inhibition TGI (%) calculated by the following formula. At the end of the experiment, at the study endpoint, or when the tumor volume reached 1500 $mm^3$, the animals were sacrificed by $CO_2$ anesthesia and dissected to give the tumors. The tumors were photographed. The calculation formula of the tumor volume (V) is: $V = 1/2 \times a \times b^2$, wherein a and b represent the length and the width respectively; $T/C(\%) = (T - T_0)/(C - Co) \times 100$, wherein T and C are the tumor volumes of the treatment group and the isotype control group at the end of the experiment, and To and Co are the tumor volumes of the treatment group and the isotype control group at the beginning of the experiment; tumor growth inhibition (TGI) (%) = 100 - T/C(%).

The results are shown in Table 6. hu5G11-hIgG1-TGF-β RII (at 3.7 and 12.3 mg/kg) has obvious inhibitory effects on the growth of MC38/hPD-L1 subcutaneous graft tumors with a tumor growth inhibition of 56% and 69% respectively, indicating a dosage dependence. The tumor growth inhibition of IgG1-TGF-β RII (2.3 mg/kg) to MC38/hPD-L1 subcutaneous graft tumors was 4%, and no significant efficacy was observed. Among them, the doses of the IgG1-TGF-β RII (2.3 mg/kg) and the hu5G11-hIgG1-TGF-β RII (3.7 mg/kg) were of the same order of magnitude and had no substantial difference. This confirms that the fusion protein of the present invention exhibits significantly higher tumor growth inhibition relative to fusion proteins having other compositions (i.e., fusion proteins comprising IgG1 and TGF-β RII). The tumor-bearing mice can well tolerate the above drug, without obvious symptoms such as weight loss.

Table 6. Efficacy of test samples on subcutaneous graft tumors of colon cancer cells MC38/hPD-L1

| Grouping | Mean tumor volume ($mm^3$) D0 | Mean tumor volume ($mm^3$) D17 | T/C (%) D17 | Tumor growth inhibition (%) D17 | P value | Number of animals per group (at beginning of study) | Number of animals per group (at end of study) |
|---|---|---|---|---|---|---|---|
| IgG1 12.3mg/kg | 57.3±0.8 | 3344.4±333.0 | - | - | - | 10 | 10 |
| hu5G11-hIgG1-TGFβ RII 3.7 mg/kg | 56.8±0.6 | 1513.1±365.8 | 44 | 56 | 0.002 | 10 | 10 |
| hu5G11-hIgG1-TGFβ RII 12.3 mg/kg | 58.1±1.9 | 1061.3±330.7 | 31 | 69 | <0.001 | 10 | 10 |
| IgG1-TGFβ RII 2.3mg/kg | 57.6±0.6 | 3219.2±279.0 | 96 | 4 | 0.776 | 10 | 10 |

Note: The animals were randomized, the time for first administration was D0, and the treatment was given by intravenous injection (IV) once every two days in a total of 6 doses. P < 0.05, P < 0.01, P < 0.001 as compared to the IgG1 group (the isotype control) by T-test.

Example 8 Cytokine Secretion Stimulated by Fusion Protein hu5G11-hIgG1-TGF-β RII Measured by Electrochemiluminescence

1) Liquid-phase method: PBMCs were adjusted with an RPMI1640 complete medium to a concentration of about 1-2 $\times 10^6$ cells/mL, and then added to a 96-well cell culture plate at 100 μL/well. IgG1 (SEQ ID NO: 38 and SEQ ID NO: 39, see Example 7 for the preparation method), LPS (SIGMA, L4391-1MG), and hu5G11-hIgG1-TGF-β RII were diluted with an RPMI1640 complete medium to give a 1000 μg/mL IgG 1 solution, a 10 μg/mL of LPS solution, and 100 μg/mL, 300 μg/mL and 1000 μg/mL of hu5G11-hIgG1-TGF-β RII solutions. The RPMI1640 complete medium was used as a negative control. 100 μL of prepared solutions was added into the 96-well cell culture plate and well mixed. The cells were cultured in a cell incubator with 5% $CO_2$ at 37 °C. The cell supernatant was collected from the 96-well plate at 24 h and 48 h. The contents of cytokines IL-2, IL-4, IL-6, TNF-α and IFN-γ were measured using a V-PLEX Proinflammatory Panel 1 (human) kit (MSD, K15049D-2). The results are shown in Table 7.

2) Solid-phase method: IgG1 (SEQ ID NO: 38 and SEQ ID NO: 39, see Example 7 for the preparation method), LPS (SIGMA, L4391-1MG), and hu5G11-hIgG1-TGF-β RII were diluted with PBS to give a 500 μg/mL IgG 1 solution, a 5 μg/mL of LPS solution, and 50 μg/mL, 150 μg/mL and 500 μg/mL of hu5G11-hIgG1-TGF-β RII solutions; PBS was used as the negative control. The prepared solutions described above were added to the corresponding wells of the 96-well high-adsorption plate at 200 μL/well for coating at 37 °C for 2 h. The supernatant was discarded, and the cell culture plate was washed 3 times with PBS. PBMCs were adjusted with an RPMI1640 complete medium to a concentration of about 1-2 $\times 10^6$ cells/mL, added to a 96-well cell culture plate at 200 μL/well and incubated in a cell incubator at 37 °C with 5% $CO_2$. The cell supernatant was collected from the 96-well plate at 24 h and 48 h. The contents of cytokines IL-2, IL-4, IL-6, TNF-α and IFN-γ were measured using a V-PLEX Proinflammatory Panel 1 (human) kit (MSD, K15049D-2). The results are shown in Table 8.

Table 7. The results of cytokine secretion stimulated by each test sample detected by liquid phase method

| Grouping | Sampling points (h) | IL-2 (pg/ml) | IL-4 (pg/ml) | IL-6 (pg/ml) | TNF-α (pg/ml) | IFN-γ (pg/ml) |
|---|---|---|---|---|---|---|
| RPMI1640 complete medium | 24 | 104.6±137.7 | 1.3±0.8 | 34.4±22.3 | 14.0±7.6 | 25.4±9.7 |
| | 48 | 426.7±621.7 | 1.1±1.0 | 30.8±34.7 | 23.6±22.7 | 496.0±174.5 |
| IgG1-1000μg/mL | 24 | 164.2±253.0 | 4.9±0.4 | 623.0±540.1 | 106.8±70.6 | 93.7±39.2 |
| | 48 | 226.0±280.4 | 6.3±2.3 | 2160.1±3028.8 | 206.3±229.7 | 1663.6±1361.7 |
| LPS-10μg/mL | 24 | 84.1±59.8 | 36.9±4.0 | 43920.0±0 | 10357.2±3119.2 | 2920.0±0 |
| | 48 | 57.3±19.3 | 43.3±3.8 | 43920.0±0 | 6254.8±2317.6 | 2920.0±0 |
| hu5G11-hIgG1-TGFβ RII-1000μg/ml | 24 | 193.2±288.5 | 2.8±0.8 | 203.0±24.1 | 68.9±13.6 | 82.1±52.3 |
| | 48 | 347.2±531.1 | 2.1±1.7 | 215.3±39.0 | 82.2±38.4 | 1588.6±314.2 |
| hu5G11-hIgG1-TGFβ RII-300μg/ml | 24 | 139.7±126.3 | 3.6±1.8 | 303.5±237.0 | 62.0±50.1 | 54.4±20.4 |
| | 48 | 357.9±484.2 | 4.0±1.7 | 485.6±341.9 | 64.4±27.3 | 839.6±667.8 |
| hu5G11-hIgG1-TGFβ RII-100μg/ml | 24 | 144.4±225.9 | 2.1±1.2 | 127.2±40.2 | 25.6±3.9 | 54.3±57.0 |
| | 48 | 315.4±407.4 | 2.7±1.4 | 195.1±67.5 | 40.2±18.6 | 1229.9±1466.5 |

Table 8. The results of cytokine secretion stimulated by each test sample detected by solid phase method

| Grouping | Sampling time (h) | IL-2 (pg/ml) | IL-4 (pg/ml) | IL-6 (pg/ml) | TNF-α (pg/ml) | IFN-γ (pg/ml) |
|---|---|---|---|---|---|---|
| PBS | 24 | 113.3±150.1 | 1.0±0.9 | 62.6±20.2 | 27.9±7.4 | 40.2±7.1 |
| | 48 | 281.7±384.2 | 1.2±0.7 | 99.0±79.8 | 43.7±17.4 | 516.4±248.8 |
| IgG1-500μg/mL | 24 | 86.8±75.2 | 6.1±0.2 | 894.3±407.1 | 955.5±562.2 | 58.0±24.2 |
| | 48 | 210.2±202.7 | 6.1±0.4 | 891.0±287.5 | 808.3±292.7 | 435.4±630.9 |
| LPS-5μg/mL | 24 | 49.7±15.3 | 48.8±12.1 | 43920.0±0 | 10700.0±3719.3 | 2920.0±0 |
| | 48 | 43.7±1.4 | 57.7±9.2 | 43920.0±0 | 10658.6±4817.8 | 2920.0±0 |
| hu5G11-hIgG1-TGFβ RII -500μg/ml | 24 | 140.2±141.7 | 7.1±1.1 | 1769.6±1294.3 | 1125.5±1122.7 | 162.1±142.0 |
| | 48 | 308.7±359.2 | 6.8±1.0 | 965.9±229.7 | 701.9±541.8 | 716.8±1063.5 |
| hu5G11-hIgG1-TGFβ RII -150μg/ml | 24 | 136.3±157.4 | 6.0±1.8 | 1747.8±2238.3 | 1118.1±1379.8 | 195.0±260.2 |
| | 48 | 240.9±263.4 | 5.4±0.8 | 525.7±216.5 | 459.3±317.3 | 644.3±835.4 |
| hu5G11-hIgG1-TGFβ RII -50μg/ml | 24 | 160.2±193.3 | 6.5±2.1 | 1266.4±1669.4 | 842.1±1063.9 | 161.2±177.0 |
| | 48 | 393.6±454.6 | 6.0±1.1 | 430.3±243.6 | 507.3±417.0 | 539.7±678.8 |

As can be seen from the results in the above table, the fusion protein of the present application exhibits a low probability of causing a cytokine storm. Therefore, the subject substantially has no risk of systemic inflammation caused by overactivating the immune system after administration.

Example 9 Toxicity Assay of Fusion Protein in Cynomolgus Monkeys

The anal temperature was detected using a Temp-14 thermistor thermometer. The respiratory rate and an II-lead electrocardiogram (ECG) were detected using noninvasive physiological signal telemetry system for large animals. The blood pressure (the systolic pressure, the diastolic pressure, and the mean arterial pressure) was measured using an noninvasive sphygmomanometer. An electrocardiogram waveform was qualitatively analyzed, and the stable and continuous electrocardiogram within a 30-second period at each time point is analyzed using ecgAUTO software. The parameters included heart rate, R-R interval, P-wave time, P-R interval, QRS wave time, Q-T interval and corrected Q-T interval.

Single-dose toxicity: In this test, 6 cynomolgus monkeys were divided into two groups of 3, including male and female. The monkeys were infused intravenously with a single dose of 300 or 1000 mg/kg of hu5G11-hIgG1-TGF-β RII, and observed for 14 days. During the test, indicators such as general observation, body weight, food intake, body temperature, II-lead ECG and blood pressure, hematology, blood biochemistry, and urine were detected. Gross anatomical observation was performed at the end of test. No death or near-death was observed during the test, and no abnormality was found in all indicators.

Repeat-dose toxicity: In this test, 40 cynomolgus monkeys were divided into 4 groups of 10, i.e., the blank control group and the treatment groups receiving 20, 60 and 200 mg/kg of hu5G11-hIgG1-TGF-β RII, half male and half female. The drug was administered once a week for 4 weeks (5 doses in total). After discontinuation, the monkeys were observed for 6 weeks during recovery. Toxicokinetic parameters of the repeated intravenous injection toxicity study are shown in Table 5 of Example 6.

At the end of treatment, the 60 mg/kg and 200 mg/kg treatment groups showed mild anemia that mainly featured decreased RBC, HGB and HCT, and increased RET and RET%. The 200 mg/kg treatment group also showed mild increase in the proportions of erythron and orthochromatic normoblasts in bone marrow smears. Histopathological examination showed that the bone marrow and the spleen had no relevant changes. By the end of the recovery period, the HGB and HCT in one female monkey in the 200 mg/kg treatment group still had no obvious recovery, and examination of the red blood cell morphology showed a decrease in hemoglobin content. The indicators of other cynomolgus monkeys described above all recovered to different extents.

At the end of treatment, slight mononuclear cell infiltration in thyroid gland was seen in the 20, 60 and 200 mg/kg treatments groups, slight to mild mononuclear cell infiltration in the kidney was seen in the 60 and 200 mg/kg treatment groups, while slight mononuclear cell infiltration in the meninx was seen in the 20 and 200 mg/kg treatment groups. By the end of the recovery period, except that the meningeal lesion in the 200 mg/kg treatment group did not recover, the organ lesions of other cynomolgus monkeys showed certain degrees of recovery.

In addition, no obvious abnormal change was observed in the cynomolgus monkeys of all the groups in the aspect of general observation, body weight, food intake, body temperature, II-lead ECG, respiratory rate and blood pressure, blood biochemistry, urine, ophthalmologic examination, bone marrow smear, immunoglobulin, complement, circulating immune complex, lymphocyte subpopulation and cytokines.

As can be seen, the fusion protein of the present application shows low acute toxicity and long-term toxicity. Therefore, it is expected that the fusion protein will show good safety in clinic use.

According to the content disclosed in the present application, the compositions and methods of the present application have been described in terms of preferred embodiments. However, it will be apparent to those skilled in the art that variations may be applied to the compositions and/or methods and the steps or the sequence of steps of the methods described herein without departing from the concept, spirit and scope of the present application. The disclosed contents of all documents cited herein are hereby incorporated by reference to the extent that they provide exemplary, procedural and other details supplementary to those described herein.

## Claims

**1.** A protein comprising: (a) at least one heavy chain variable domain and at least one light chain variable domain of an antibody binding to human programmed death-ligand 1 (PD-L1); and (b) human TGF-β RII or a TGF-β binding fragment thereof,
Wherein, in the antibody, a heavy chain CDR1 sequence comprises an amino acid sequence set forth in SEQ ID NO: 1 or SEQ ID NO: 15, a heavy chain CDR2 sequence comprises an amino acid sequence set forth in SEQ ID NO: 2 or SEQ ID NO: 16, and a heavy chain CDR3 sequence comprises an amino acid sequence set forth in SEQ ID NO: 3 or SEQ ID NO: 17; and a light chain CDR1 sequence comprises an amino acid sequence set forth in SEQ ID NO: 4 or SEQ ID NO: 18, a light chain CDR2 sequence comprises an amino acid sequence set forth in SEQ ID NO: 5 or SEQ ID NO: 19, and a light chain CDR3 sequence comprises an amino acid sequence set forth in SEQ ID NO: 6 or SEQ ID NO: 20.

**2.** The protein according to claim 1, wherein the heavy chain variable domain comprises an amino acid sequence set forth in SEQ ID NO: 7 or SEQ ID NO: 21, and the light chain variable domain comprises an amino acid sequence set forth in SEQ ID NO: 8 or SEQ ID NO: 22.

**3.** The protein according to claim 1 or 2, wherein the heavy chain variable domain comprises an amino acid sequence set forth in SEQ ID NO: 7, and the light chain variable domain comprises an amino acid sequence set forth in SEQ ID NO: 8.

**4.** The protein according to any one of claims 1-3, wherein the heavy chain variable domain comprises an amino acid sequence set forth in SEQ ID NO: 21, and the light chain variable domain comprises an amino acid sequence set forth in SEQ ID NO: 22.

**5.** The protein according to any one of claims 1-4, wherein the antibody comprises: a heavy chain amino acid sequence having at least 95% identity to an amino acid sequence set forth in SEQ ID NO: 9, SEQ ID NO: 23, SEQ ID NO: 29 or SEQ ID NO: 32; and a light chain amino acid sequence having at least 95% identity to an amino acid sequence set forth in SEQ ID NO: 10 or SEQ ID NO: 24.

**6.** The protein according to any one of claims 1-5, wherein the human TGF-β RII or the TGF-β binding fragment thereof comprises an amino acid sequence having at least 80% identity to an amino acid sequence set forth in SEQ ID NO: 36.

**7.** The protein according to any one of claims 1-6, wherein the protein further comprises a linker peptide linking a C terminus of the antibody or the antigen binding fragment thereof to an N terminus of the human TGF-β RII or the TGF-β binding fragment thereof.

**8.** The protein according to claim 7, wherein the linker peptide is $(G_4S)_xG$, and x is an integer of 3-6; preferably the linker peptide has an amino acid sequence set forth in SEQ ID NO: 37.

**9.** A polynucleotide sequence encoding the protein according to any one of claims 1-8.

**10.** The polynucleotide sequence according to claim 9, wherein the polynucleotide sequence comprises: a nucleic acid sequence set forth in SEQ ID NO: 12, a nucleic acid sequence set forth in SEQ ID NO: 26, a nucleic acid sequence set forth in SEQ ID NO: 31 or a nucleic acid sequence set forth in SEQ ID NO: 34, and a nucleic acid sequence set forth in SEQ ID NO: 14 or a nucleic acid sequence set forth in SEQ ID NO: 28; preferably, the polynucleotide sequence comprises: the nucleic acid sequences set forth in SEQ ID NO: 34 and SEQ ID NO: 28, or the nucleic acid sequences set forth in SEQ ID NO: 31 and SEQ ID NO: 14.

**11.** An expression vector comprising the polynucleotide sequence according to claim 9 or 10.

**12.** A cell comprising the polynucleotide sequence according to claim 9 or 10 or the expression vector according to claim 11.

**13.** A method for treating cancer, inhibiting tumor growth, or enhancing an anti-tumor response, comprising administering to a subject in need thereof the protein according to any one of claims 1-8.

**14.** The method according to claim 13, wherein the protein is administered as monotherapy or in combination with radiotherapy, chemotherapy, surgery, biologics, or chemicals.

**15.** The method according to any one of claims 13-14, wherein the cancer or tumor is selected from the group consisting of: lung adenocarcinoma, mucinous adenocarcinoma, low-grade brain neuroglioma, glioblastoma multiforme, mesothelioma, melanoma, thyroid cancer, renal cancer, liver cancer, acute myeloid leukemia, esophageal adenocarcinoma, lymphoma, non-small cell lung cancer, metastatic non-small cell lung cancer, advanced or recurrent non-small cell lung cancer, refractory non-small cell lung cancer after chemotherapy, metastatic non-squamous non-small cell lung cancer, advanced or recurrent non-squamous non-small cell lung cancer, unresectable advanced non-small cell lung cancer, occult non-small cell lung cancer, breast cancer, metastatic breast cancer, triple-negative breast cancer, advanced or recurrent breast cancer, locally recurrent breast cancer, inflammatory breast cancer, pancreatic ductal adenocarcinoma, metastatic pancreatic cancer, locally advanced unresectable pancreatic cancer, recurrent pancreatic cancer, prostate cancer, advanced or metastatic prostate cancer, locally advanced prostate cancer, castration-resistant prostate cancer, recurrent prostate cancer after castration, localized prostate cancer, progressive prostate cancer, colorectal cancer, rectal adenocarcinoma, large intestinal cancer, metastatic colorectal cancer, advanced or recurrent colon cancer, advanced or recurrent rectal cancer, locally recurrent rectal cancer, locally recurrent colon cancer, gastric adenocarcinoma, gastric cancer, unresectable gastric cancer, metastatic gastric cancer, locally advanced or recurrent gastric cancer, gastrointestinal stromal tumor, biliary tract cancer, bile duct cancer, gallbladder cancer, unresectable or metastatic biliary tract cancer, unresectable or metastatic bile duct cancer, unresectable or metastatic gallbladder cancer, penile cancer, anal cancer, vaginal cancer, cervical cancer, locally advanced cervical cancer, recurrent cervical cancer, metastatic cervical cancer, metastatic penile cancer, advanced or recurrent vaginal squamous cell carcinoma, advanced or recurrent vaginal adenocarcinoma, uterine corpus endometrioid carcinoma, bladder cancer, human papillomavirus infection, head and neck cancer, recurrent or metastatic head and neck cancer, hypopharyngeal cancer, laryngeal cancer, oral cancer, nasopharyngeal carcinoma, oropharyngeal cancer, pharyngolaryngeal cancer, paranasal sinus and nasal cavity cancer, and salivary gland cancer.

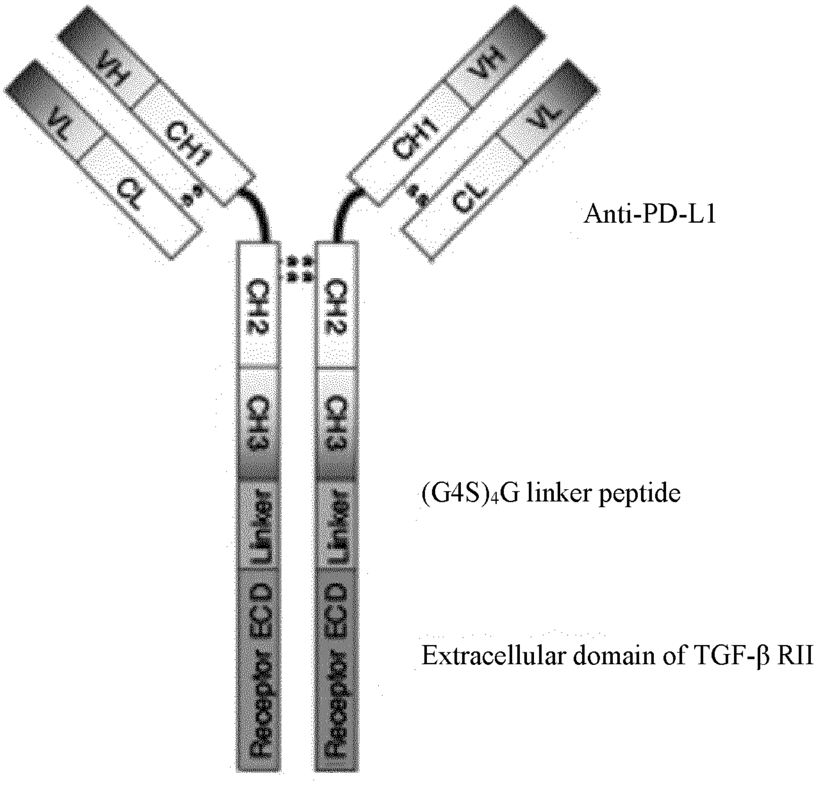
VH
CH1
VL
CL
CH2
CH3
Linker
Receptor ECD
Anti-PD-L1
(G4S)4G linker peptide
Extracellular domain of TGF-β RII

FIG. 1

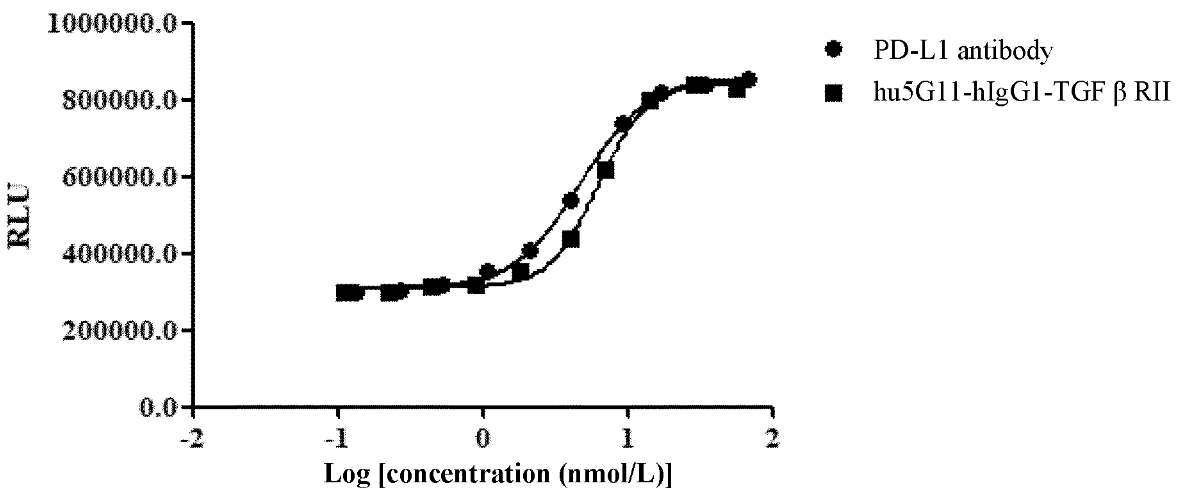
1000000.0
800000.0
600000.0
400000.0
200000.0
0.0
RLU
-2
-1
0
1
2
Log [concentration (nmol/L)]
PD-L1 antibody
hu5G11-hIgG1-TGF β RII

FIG. 2A

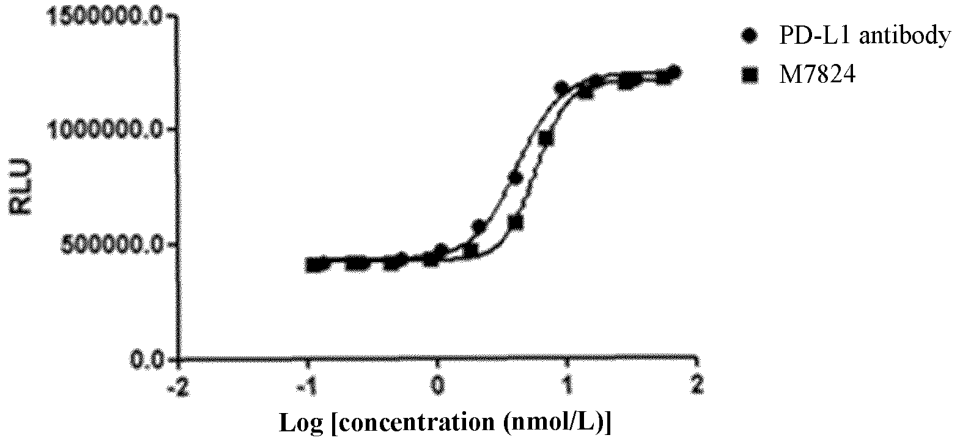


FIG. 2B

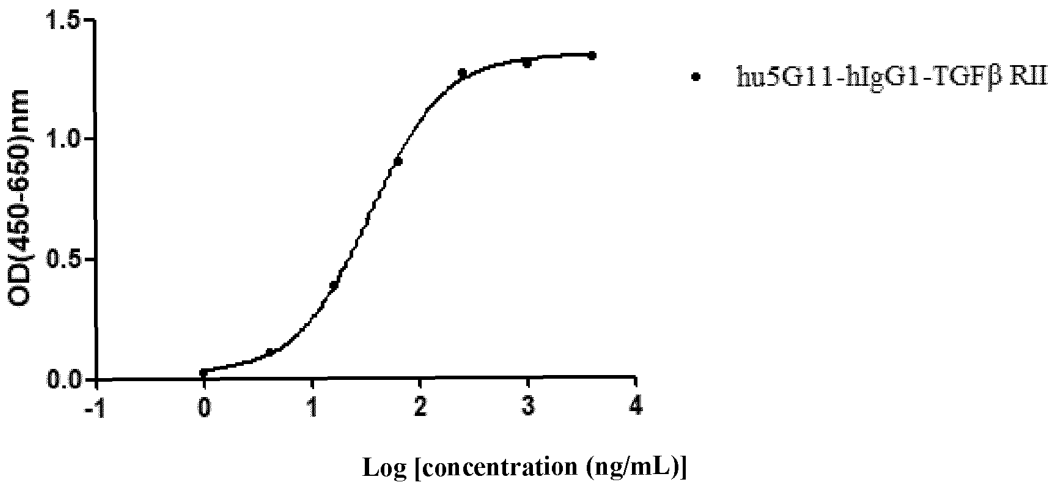


FIG. 3

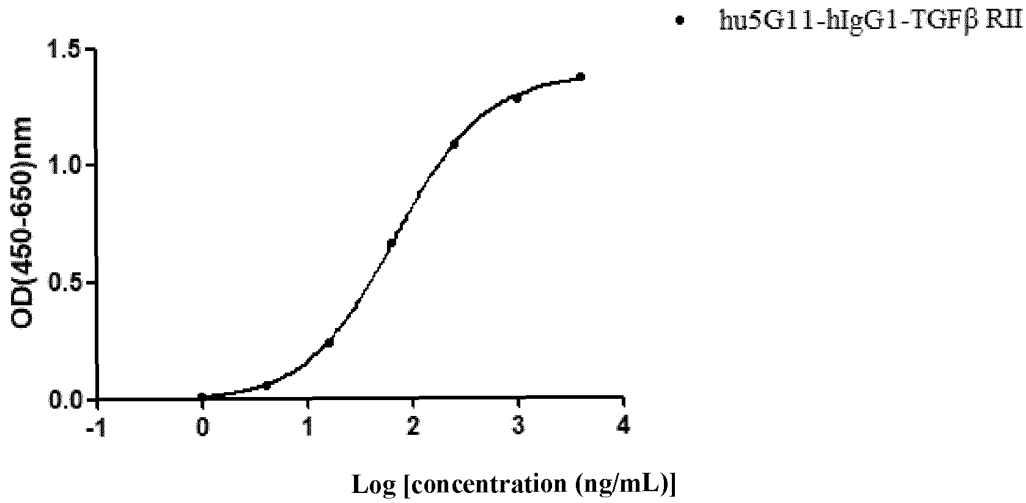


FIG. 4

## INTERNATIONAL SEARCH REPORT

International application No.
**PCT/CN2020/111983**

**A. CLASSIFICATION OF SUBJECT MATTER**

C07K 19/00(2006.01)i; C07K 14/71(2006.01)i; A61K 38/17(2006.01)i; A61P 35/00(2006.01)i; A61K 39/395(2006.01)i; C12N 15/62(2006.01)i; C12N 15/63(2006.01)i

According to International Patent Classification (IPC) or to both national classification and IPC

**B. FIELDS SEARCHED**

Minimum documentation searched (classification system followed by classification symbols)

C07K; C12N; A61K; A61P

Documentation searched other than minimum documentation to the extent that such documents are included in the fields searched

Electronic data base consulted during the international search (name of data base and, where practicable, search terms used)

CNABS, DWPI, SIPOABS, CNTXT, WOTXT, USTXT, EPTXT, CNKI, PUBMED, ISI WEB OF SCIENCE, 百度学术, BAIDU XUESHU: 程序性死亡配体1, 转化生长因子, 转化生长因子受体, PD-L1, CD247, B7-H1, TGFβ, TGFβ RII; GenBank +EMBL+中国专利生物序列检索系统, GenBank+EMBL+Chinese Patent Biological Sequence Retrieval System: 对于SEQ ID NOs.1-10, 15-24, 29, 32, 36, 37进行序列检索, sequence search on SEQ ID NOs. 1-10, 15-24, 29, 32, 36, 37。

**C. DOCUMENTS CONSIDERED TO BE RELEVANT**

| Category* | Citation of document, with indication, where appropriate, of the relevant passages | Relevant to claim No. |
|---|---|---|
| Y | WO 2018205985 A1 (JIANGSU HENGRUI MEDICINE CO., LTD. et al.) 15 November 2018 (2018-11-15)<br>see the abstract, description, page 7, line 30 to page 8, line 5, and embodiment 1 | 1-15 |
| Y | CN 107001463 A (CROWN BIOSCIENCE INC.; CHIA TAI TIANQING PHARMACEUTICAL GROUP CO., LTD.) 01 August 2017 (2017-08-01)<br>see description, tables 1-3 | 1-15 |
| Y | CN 106103488 A (MERCK PATENT GMBH) 09 November 2016 (2016-11-09)<br>see claims 1-25 | 1-15 |
| Y | WO 2011109789 A2 (JOHNS HOPKINS UNIVERSITY et al.) 09 September 2011 (2011-09-09)<br>see claims 1-8 | 1-15 |
| Y | CN 107460221 A (CHIA TAI TIANQING PHARMACEUTICAL GROUP CO., LTD.) 12 December 2017 (2017-12-12)<br>see claims 1-16 | 1-15 |

☑ Further documents are listed in the continuation of Box C. ☑ See patent family annex.

* Special categories of cited documents:
"A" document defining the general state of the art which is not considered to be of particular relevance
"E" earlier application or patent but published on or after the international filing date
"L" document which may throw doubts on priority claim(s) or which is cited to establish the publication date of another citation or other special reason (as specified)
"O" document referring to an oral disclosure, use, exhibition or other means
"P" document published prior to the international filing date but later than the priority date claimed
"T" later document published after the international filing date or priority date and not in conflict with the application but cited to understand the principle or theory underlying the invention
"X" document of particular relevance; the claimed invention cannot be considered novel or cannot be considered to involve an inventive step when the document is taken alone
"Y" document of particular relevance; the claimed invention cannot be considered to involve an inventive step when the document is combined with one or more other such documents, such combination being obvious to a person skilled in the art
"&" document member of the same patent family

| Date of the actual completion of the international search | Date of mailing of the international search report |
|---|---|
| **24 November 2020** | **09 December 2020** |

| Name and mailing address of the ISA/CN | Authorized officer |
|---|---|
| **China National Intellectual Property Administration (ISA/CN)**<br>**No. 6, Xitucheng Road, Jimenqiao Haidian District, Beijing 100088**<br>**China** | |
| Facsimile No. **(86-10)62019451** | Telephone No. |

Form PCT/ISA/210 (second sheet) (January 2015)

**INTERNATIONAL SEARCH REPORT**

International application No.
**PCT/CN2020/111983**

**C. DOCUMENTS CONSIDERED TO BE RELEVANT**

| Category* | Citation of document, with indication, where appropriate, of the relevant passages | Relevant to claim No. |
|---|---|---|
| A | CN 108883180 A (ORIONIS BIOSCIENCES NV; VIB VZW; GHENT UNIVERSITY) 23 November 2018 (2018-11-23)<br>see entire document | 1-15 |
| A | WO 2018141964 A1 (ORIONIS BIOSCIENCES N.V. et al.) 09 August 2018 (2018-08-09)<br>see entire document | 1-15 |
| Y | RAVI R. et al. "Bifunctional Immune Checkpoint-targeted Antibody-ligand Traps That Simultaneously Disable TGFβ Enhance the Efficacy of Cancer Immunotherapy"<br>*Nature Communications,* Vol. 9, No. 1, 21 February 2018 (2018-02-21),<br>ISSN: 2041-1723,<br>pp. 1-14, see the abstract, and figure 2 | 1-15 |
| Y | GRENGA I. et al. "Anti-PD-L1/TGFβR2 (M7824) Fusion Protein Induces Immunogenic Modulation of Human Urothelial Carcinoma Cell Lines, Rendering Them More Susceptible to Immune-mediated Recognition and Lysis"<br>*Urologic Oncology,* Vol. 36, No. 3, 31 March 2018 (2018-03-31),<br>ISSN: 1873-2496,<br>pp. 1-21, and see the abstract | 1-15 |
| Y | 胡梦雪等 (HU, Mengxue et al.). "PD-L1/TGF-β双功能抑制剂融合蛋白M7824研究进展 (Advances of Bifunctional Anti-PD-L1/TGF-β Fusion Protein M7824)"<br>*国际肿瘤学杂志 (Journal of International Oncology),*<br>Vol. 46, No. 5, 31 May 2019 (2019-05-31),<br>ISSN: 1673-422X,<br>pp. 281-284, and see the abstract | 1-15 |

Form PCT/ISA/210 (second sheet) (January 2015)

**INTERNATIONAL SEARCH REPORT**

International application No.
**PCT/CN2020/111983**

**Box No. II Observations where certain claims were found unsearchable (Continuation of item 2 of first sheet)**

This international search report has not been established in respect of certain claims under Article 17(2)(a) for the following reasons:

1. ☑ Claims Nos.: **13-15**
because they relate to subject matter not required to be searched by this Authority, namely:

[1] The subject matter of claims 13-15 is "a method for treating cancer, known tumor growth or enhancing anti-tumor response". The technical scheme is a treatment method for diseases of human or animal bodies, and does not warrant an international search according to the criteria set out in PCT Rule 39.1(iv).

[2] The search and opinion on claims 13-15 are made based on the reasonably anticipated modified subject matter "use of the protein according to any one of claims 1-8 in the preparation of a drug for treating cancer, inhibiting tumor growth or enhancing tumor response".

2. ☐ Claims Nos.:
because they relate to parts of the international application that do not comply with the prescribed requirements to such an extent that no meaningful international search can be carried out, specifically:

3. ☐ Claims Nos.:
because they are dependent claims and are not drafted in accordance with the second and third sentences of Rule 6.4(a).

**INTERNATIONAL SEARCH REPORT**
**Information on patent family members**

International application No.
**PCT/CN2020/111983**

| Patent document cited in search report | Publication date (day/month/year) | Patent family member(s) | Publication date (day/month/year) |
|---|---|---|---|
| WO 2018205985 A1 | 15 November 2018 | KR 20200004801 A | 14 January 2020 |
| | | EP 3623389 A1 | 18 March 2020 |
| | | JP 2020519289 A | 02 July 2020 |
| | | AU 2018264455 A1 | 14 November 2019 |
| | | US 2020157180 A1 | 21 May 2020 |
| | | MX 2019013023 A | 18 December 2019 |
| | | TW 201900674 A | 01 January 2019 |
| | | BR 112019023184 A2 | 19 May 2020 |
| | | CN 110050000 A | 23 July 2019 |
| | | CA 3061791 A1 | 29 October 2019 |
| CN 107001463 A | 01 August 2017 | SG 11201700687T A | 27 February 2017 |
| | | CN 110964108 A | 07 April 2020 |
| | | EP 3177649 A1 | 14 June 2017 |
| | | CN 107001463 B | 17 January 2020 |
| | | RU 2017106804 A | 06 September 2018 |
| | | US 2017204184 A1 | 20 July 2017 |
| | | JP 2017523786 A | 24 August 2017 |
| | | KR 20170039706 A | 11 April 2017 |
| | | WO 2016022630 A1 | 11 February 2016 |
| | | IL 250415 D0 | 30 March 2017 |
| | | RU 2017106804 A3 | 27 August 2019 |
| | | RU 2722212 C2 | 28 May 2020 |
| | | US 10435470 B2 | 08 October 2019 |
| | | SG 10201901057U A | 28 March 2019 |
| | | MX 2017001597 A | 17 November 2017 |
| | | US 2020031935 A1 | 30 January 2020 |
| | | CN 110964109 A | 07 April 2020 |
| | | CA 2956399 A1 | 11 February 2016 |
| | | BR 112017002234 A2 | 24 July 2018 |
| | | RU 2722212 C9 | 23 July 2020 |
| | | AU 2015301126 A1 | 23 February 2017 |
| | | EP 3177649 A4 | 25 April 2018 |
| CN 106103488 A | 09 November 2016 | US 2018002436 A1 | 04 January 2018 |
| | | PH 12016501549 A1 | 03 October 2016 |
| | | WO 2015118175 A3 | 08 October 2015 |
| | | WO 2015118175 A2 | 13 August 2015 |
| | | US 9676863 B2 | 13 June 2017 |
| | | EP 3105246 A2 | 21 December 2016 |
| | | RU 2016135062 A3 | 12 December 2018 |
| | | CA 2934979 A1 | 13 August 2015 |
| | | SG 11201606577Y A | 29 September 2016 |
| | | PE 20161096 A1 | 22 October 2016 |
| | | JP 6731346 B2 | 29 July 2020 |
| | | AU 2019246876 A1 | 31 October 2019 |
| | | JP 2017506217 A | 02 March 2017 |
| | | IL 246968 D0 | 29 September 2016 |
| | | RU 2016135062 A | 15 March 2018 |
| | | AU 2015213988 B2 | 11 July 2019 |
| | | MX 2016010067 A | 07 October 2016 |
| | | US 2020055949 A1 | 20 February 2020 |

Form PCT/ISA/210 (patent family annex) (January 2015)

**INTERNATIONAL SEARCH REPORT**
**Information on patent family members**

International application No.
**PCT/CN2020/111983**

| Patent document cited in search report | Publication date (day/month/year) | Patent family member(s) | Publication date (day/month/year) |
|---|---|---|---|
| | | CL 2016002017 A1 | 09 June 2017 |
| | | US 2015225483 A1 | 13 August 2015 |
| | | KR 20160119197 A | 12 October 2016 |
| | | AU 2015213988 A1 | 07 July 2016 |
| WO 2011109789 A2 | 05 April 2012 | PL 2542590 T5 | 10 August 2020 |
| | | ES 2638521 T3 | 23 October 2017 |
| | | JP 2018172439 A | 08 November 2018 |
| | | EP 2542590 A2 | 09 January 2013 |
| | | US 2016340430 A1 | 24 November 2016 |
| | | JP 6066732 B2 | 25 January 2017 |
| | | PL 2542590 T3 | 30 November 2017 |
| | | IL 221607 A | 31 May 2020 |
| | | EP 2542590 A4 | 31 July 2013 |
| | | US 2020115455 A1 | 16 April 2020 |
| | | US 9850306 B2 | 26 December 2017 |
| | | PT 2542590 T | 31 August 2017 |
| | | DK 2542590 T4 | 13 July 2020 |
| | | WO 2011109789 A2 | 09 September 2011 |
| | | DK 2542590 T3 | 28 August 2017 |
| | | US 2015183881 A1 | 02 July 2015 |
| | | US 9441044 B2 | 13 September 2016 |
| | | US 8993524 B2 | 31 March 2015 |
| | | EP 2542590 B1 | 03 May 2017 |
| | | US 10442860 B2 | 15 October 2019 |
| | | JP 2017043600 A | 02 March 2017 |
| | | IL 266971 D0 | 31 July 2019 |
| | | JP 2013521311 A | 10 June 2013 |
| | | EP 2542590 B2 | 01 April 2020 |
| | | CA 3083324 A1 | 09 September 2011 |
| | | US 2013039911 A1 | 14 February 2013 |
| | | US 2017158770 A1 | 08 June 2017 |
| | | JP 6378262 B2 | 22 August 2018 |
| | | EP 3260470 A1 | 27 December 2017 |
| | | CA 2791383 A1 | 09 September 2011 |
| CN 107460221 A | 12 December 2017 | None | |
| CN 108883180 A | 23 November 2018 | CA 3013551 A1 | 10 August 2017 |
| | | CN 109071663 A | 21 December 2018 |
| | | CN 109071627 A | 21 December 2018 |
| | | EP 3411407 A1 | 12 December 2018 |
| | | EP 3411065 A1 | 12 December 2018 |
| | | WO 2017134301 A1 | 10 August 2017 |
| | | CN 109071632 A | 21 December 2018 |
| | | CA 3013554 A1 | 10 August 2017 |
| | | JP 2019506867 A | 14 March 2019 |
| | | WO 2017134302 A2 | 10 August 2017 |
| | | CA 3013555 A1 | 10 August 2017 |
| | | WO 2017134302 A3 | 28 September 2017 |
| | | JP 2019506869 A | 14 March 2019 |
| | | WO 2017134305 A1 | 10 August 2017 |
| | | US 2019352406 A1 | 21 November 2019 |

Form PCT/ISA/210 (patent family annex) (January 2015)

**INTERNATIONAL SEARCH REPORT**
**Information on patent family members**

International application No.
**PCT/CN2020/111983**

| Patent document cited in search report | Publication date (day/month/year) | Patent family member(s) | Publication date (day/month/year) |
|---|---|---|---|
| | | US 2019367604 A1 | 05 December 2019 |
| | | WO 2017134306 A1 | 10 August 2017 |
| | | CA 3013558 A1 | 10 August 2017 |
| | | JP 2019510478 A | 18 April 2019 |
| | | EP 3411398 A2 | 12 December 2018 |
| | | US 2019071500 A1 | 07 March 2019 |
| | | JP 2019507135 A | 14 March 2019 |
| | | EP 3411397 A1 | 12 December 2018 |
| WO 2018141964 A1 | 09 August 2018 | CN 110546160 A | 06 December 2019 |
| | | US 2020087411 A1 | 19 March 2020 |
| | | KR 20190115005 A | 10 October 2019 |
| | | EP 3577133 A1 | 11 December 2019 |
| | | IL 268346 D0 | 26 September 2019 |
| | | JP 2020506727 A | 05 March 2020 |
| | | CA 3052523 A1 | 09 August 2018 |
| | | AU 2018216032 A1 | 08 August 2019 |
| | | MX 2019009255 A | 05 November 2019 |

Form PCT/ISA/210 (patent family annex) (January 2015)

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- CN 106103488
- CN 107001463

Sequence Listing

<110>  NANJING SHUNXIN PHARMACEUTICALS CO., LTD. OF CHIATAI TIANQING PHARMACEUTICAL GROUP
        CHIATAI TIANQING PHARMACEUTICAL GROUP CO., LTD.

<120>  FUSION PROTEIN TARGETING PD-L1 AND TGF-β AND USE THEREOF

<130>  2019

<150>  201910815301.2
<151>  2019-08-30

<160>  43

<170>  SIPOSequenceListing 1.0

<210>  1
<211>  5
<212>  PRT
<213>  Mus sp.

<400>  1
Ser Tyr Gly Met Ser
1               5

<210>  2
<211>  16
<212>  PRT
<213>  Mus sp.

<400>  2
Ser Ile Ser Ser Gly Gly Ser Thr Tyr Tyr Pro Asp Ser Val Lys Gly
1               5                   10                  15

<210>  3
<211>  8
<212>  PRT
<213>  Mus sp.

<400>  3
Gly Tyr Asp Ser Gly Phe Ala Tyr
1               5

<210>  4
<211>  14
<212>  PRT
<213>  Mus sp.

<400>  4
Ala Ser Gln Ser Val Ser Thr Ser Ser Ser Ser Phe Met His
1               5                   10

<210>  5
<211>  7
<212>  PRT
<213>  Mus sp.

```
<400>  5
Tyr Ala Ser Asn Leu Glu Ser
1               5

<210>  6
<211>  9
<212>  PRT
<213>  Mus sp.

<400>  6
Gln His Ser Trp Glu Ile Pro Tyr Thr
1               5

<210>  7
<211>  116
<212>  PRT
<213>  Synthetic sequence

<220>
<223>  Heavy chain variable domain of PD-L1 antibody

<400>  7
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Ile Phe Arg Ser Tyr
            20                  25                  30
Gly Met Ser Trp Val Arg Gln Ala Pro Gly Lys Gly Leu Glu Trp Val
        35                  40                  45
Ala Ser Ile Ser Ser Gly Gly Ser Thr Tyr Tyr Pro Asp Ser Val Lys
    50                  55                  60
Gly Arg Phe Thr Ile Ser Arg Asp Asn Ala Lys Asn Ser Leu Tyr Leu
65                  70                  75                  80
Gln Met Asn Ser Leu Arg Ala Glu Asp Thr Ala Val Tyr Asp Cys Ala
                85                  90                  95
Arg Gly Tyr Asp Ser Gly Phe Ala Tyr Trp Gly Gln Gly Thr Leu Val
            100                 105                 110
Thr Val Ser Ser
        115

<210>  8
<211>  111
<212>  PRT
<213>  Synthetic sequence

<220>
<223>  Light chain variable domain of PD-L1 antibody

<400>  8
Asp Ile Val Leu Thr Gln Ser Pro Ala Ser Leu Ala Val Ser Pro Gly
1               5                   10                  15
Gln Arg Ala Thr Ile Thr Cys Arg Ala Ser Gln Ser Val Ser Thr Ser
            20                  25                  30
Ser Ser Ser Phe Met His Trp Tyr Gln Gln Lys Pro Gly Gln Pro Pro
        35                  40                  45
Lys Leu Leu Ile Lys Tyr Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
```

Pro Val Glu Ala Asn Asp Thr Ala Asn Tyr Tyr Cys Gln His Ser Trp
85 90 95
Glu Ile Pro Tyr Thr Phe Gly Gln Gly Thr Lys Leu Glu Ile Lys
100 105 110

<210> 9
<211> 446
<212> PRT
<213> Synthetic sequence

<220>
<223> Heavy chain of PD-L1 antibody

<400> 9
Glu Val Gln Leu Val Glu Ser Gly Gly Gly Leu Val Lys Pro Gly Gly
1 5 10 15
Ser Leu Arg Leu Ser Cys Ala Ala Ser Gly Phe Ile Phe Arg Ser Tyr
20 25 30
Gly Met Ser Trp Val Arg Gln Ala Pro Gly Lys Gly Leu Glu Trp Val
35 40 45
Ala Ser Ile Ser Ser Gly Gly Ser Thr Tyr Tyr Pro Asp Ser Val Lys
50 55 60
Gly Arg Phe Thr Ile Ser Arg Asp Asn Ala Lys Asn Ser Leu Tyr Leu
65 70 75 80
Gln Met Asn Ser Leu Arg Ala Glu Asp Thr Ala Val Tyr Asp Cys Ala
85 90 95
Arg Gly Tyr Asp Ser Gly Phe Ala Tyr Trp Gly Gln Gly Thr Leu Val
100 105 110
Thr Val Ser Ser Ala Ser Thr Lys Gly Pro Ser Val Phe Pro Leu Ala
115 120 125
Pro Ser Ser Lys Ser Thr Ser Gly Gly Thr Ala Ala Leu Gly Cys Leu
130 135 140
Val Lys Asp Tyr Phe Pro Glu Pro Val Thr Val Ser Trp Asn Ser Gly
145 150 155 160
Ala Leu Thr Ser Gly Val His Thr Phe Pro Ala Val Leu Gln Ser Ser
165 170 175
Gly Leu Tyr Ser Leu Ser Ser Val Val Thr Val Pro Ser Ser Ser Leu
180 185 190
Gly Thr Gln Thr Tyr Ile Cys Asn Val Asn His Lys Pro Ser Asn Thr
195 200 205
Lys Val Asp Lys Lys Val Glu Pro Lys Ser Cys Asp Lys Thr His Thr
210 215 220
Cys Pro Pro Cys Pro Ala Pro Glu Leu Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Ala Val Ser His Glu Asp Pro Glu Val
260 265 270
Lys Phe Asn Trp Tyr Val Asp Gly Val Glu Val His Asn Ala Lys Thr
275 280 285
Lys Pro Arg Glu Glu Gln Tyr Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Ala Leu Pro Ala Pro Ile Glu Lys Thr Ile Ser
325 330 335
Lys Ala Lys Gly Gln Pro Arg Glu Pro Gln Val Tyr Thr Leu Pro Pro
340 345 350

Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445

<210> 10
<211> 218
<212> PRT
<213> Synthetic sequence

<220>
<223> Light chain of PD-L1 antibody

<400> 10
Asp Ile Val Leu Thr Gln Ser Pro Ala Ser Leu Ala Val Ser Pro Gly
1               5                   10                  15
Gln Arg Ala Thr Ile Thr Cys Arg Ala Ser Gln Ser Val Ser Thr Ser
            20                  25                  30
Ser Ser Ser Phe Met His Trp Tyr Gln Gln Lys Pro Gly Gln Pro Pro
        35                  40                  45
Lys Leu Leu Ile Lys Tyr Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Asn Asp Thr Ala Asn Tyr Tyr Cys Gln His Ser Trp
                85                  90                  95
Glu Ile Pro Tyr Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215

<210> 11
<211> 603
<212> PRT
<213> Synthetic sequence

<220>
<223> Heavy chain of anti-PD-L1-TGF-β RII fusion protein

<400> 11

Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Ile Phe Arg Ser Tyr
            20                  25                  30
Gly Met Ser Trp Val Arg Gln Ala Pro Gly Lys Gly Leu Glu Trp Val
        35                  40                  45
Ala Ser Ile Ser Ser Gly Gly Ser Thr Tyr Tyr Pro Asp Ser Val Lys
    50                  55                  60
Gly Arg Phe Thr Ile Ser Arg Asp Asn Ala Lys Asn Ser Leu Tyr Leu
65                  70                  75                  80
Gln Met Asn Ser Leu Arg Ala Glu Asp Thr Ala Val Tyr Asp Cys Ala
                85                  90                  95
Arg Gly Tyr Asp Ser Gly Phe Ala Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Ala Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys Gly Gly
        435                 440                 445

```
Gly Gly Ser Gly Gly Gly Gly Ser Gly Gly Gly Gly Ser Gly Gly Gly
    450                 455                 460
Gly Ser Gly Ile Pro Pro His Val Gln Lys Ser Val Asn Asn Asp Met
465                 470                 475                 480
Ile Val Thr Asp Asn Asn Gly Ala Val Lys Phe Pro Gln Leu Cys Lys
                485                 490                 495
Phe Cys Asp Val Arg Phe Ser Thr Cys Asp Asn Gln Lys Ser Cys Met
            500                 505                 510
Ser Asn Cys Ser Ile Thr Ser Ile Cys Glu Lys Pro Gln Glu Val Cys
        515                 520                 525
Val Ala Val Trp Arg Lys Asn Asp Glu Asn Ile Thr Leu Glu Thr Val
    530                 535                 540
Cys His Asp Pro Lys Leu Pro Tyr His Asp Phe Ile Leu Glu Asp Ala
545                 550                 555                 560
Ala Ser Pro Lys Cys Ile Met Lys Glu Lys Lys Lys Pro Gly Glu Thr
                565                 570                 575
Phe Phe Met Cys Ser Cys Ser Ser Asp Glu Cys Asn Asp Asn Ile Ile
            580                 585                 590
Phe Ser Glu Glu Tyr Asn Thr Ser Asn Pro Asp
        595                 600
```

<210> 12
<211> 1809
<212> DNA
<213> Synthetic sequence

<220>
<223> Nucleic acid sequence for encoding heavy chain of anti-PD-L1-TGF-β RII fusion protein

<400> 12

```
gaggtgcagc tggtcgagtc aggagggggg ctggtcaagc caggagggtc actgcgactg      60
agctgcgcag cttccgggtt catctttagg tcttatggca tgagttgggt gcgccaggca     120
ccagggaaag gactggagtg ggtcgcttca atcagctccg gaggcagcac ttactatcct     180
gactccgtga agggccggtt caccatttct agagataacg ccaaaaatag tctgtacctg     240
cagatgaact ctctgcgagc agaagacaca gccgtctacg attgtgctag aggatatgac     300
agcggctttg catactgggg ccaggggacc ctggtgacag tctcgagcgc ctccactaag     360
ggcccatccg tgttccctct ggcaccctcc agcaagagca caagcggagg caccgccgca     420
ctgggctgcc tcgtgaagga ctacttccca gaacccgtga ccgtcagctg gaatagcggc     480
gctctgacca gcggagtcca cactttcccc gcagtgctgc agtccagcgg cctgtacagc     540
ctgagcagcg tggtcactgt gccaagcagc agcctgggca ctcagaccta catctgcaac     600
gtcaaccaca agcccagcaa cacaaaggtg gacaagaagg tcgagcccaa gtcctgcgat     660
aagacccaca cctgccctcc atgtcccgcc cccgagctgc tgggaggacc cagcgtcttc     720
ctgtttcccc ccaagccaaa ggacaccctg atgatcagca ggacccccga agtgacctgc     780
gtcgtggtgg ccgtgagcca cgaagatccc gaggtgaagt tcaactggta cgtggacggc     840
gtggaagtgc acaacgccaa gacaaaaccc agggaggagc agtataacag cacctacagg     900
gtcgtgagcg tcctgaccgt gctgcaccaa gactggctga acggcaagga gtataagtgc     960
aaggtgagca acaaggcact gcccgccccc atcgagaaga ccatttccaa ggccaagggg    1020
caacctaggg agccacaggt ctacactctg ccccctagca gggacgagct gaccaagaac    1080
caggtctccc tgacttgcct ggtgaagggg ttttatccca gcgacatcgc cgtcgagtgg    1140
gagagcaatg gccagcccga aaacaactac aagaccacac cccctgtgct ggacagcgac    1200
ggcagcttct ttctgtatag caaactgaca gtggataaga gcagatggca gcagggcaac    1260
gtgttctcct gctccgtgat gcacgaggcc ctgcacaatc actacaccca gaagtccctg    1320
agcctgtccc ccggaaaagg aggaggagga tctggaggag gcggcagcgg cggaggagga    1380
agtggaggag gcggatccgg catccctccc cacgtgcaga agagcgtgaa taacgacatg    1440
atcgtgaccg acaataacgg cgctgtgaag ttccctcagc tgtgcaagtt ctgcgatgtg    1500
cggttctcca cctgcgacaa tcagaagagc tgcatgagca actgcagcat cacctccatc    1560
tgcgagaagc ctcaggaggt gtgtgtggcc gtgtggcgga agaatgacga gaatatcacc    1620
```

```
ctggagaccg tgtgccacga ccccaagctg ccttatcacg atttcatcct ggaggacgcc      1680
gctagcccca agtgcatcat gaaggagaag aagaagcccg gcgagacctt cttcatgtgt      1740
agctgtagca gcgatgagtg caacgataat atcatcttta gcgaggagta taacaccagc      1800
aatcccgat                                                              1809
```

<210> 13
<211> 218
<212> PRT
<213> Synthetic sequence

<220>
<223> Light chain of anti-PD-L1-TGF-β RII fusion protein

<400> 13

```
Asp Ile Val Leu Thr Gln Ser Pro Ala Ser Leu Ala Val Ser Pro Gly
1               5                   10                  15
Gln Arg Ala Thr Ile Thr Cys Arg Ala Ser Gln Ser Val Ser Thr Ser
            20                  25                  30
Ser Ser Ser Phe Met His Trp Tyr Gln Gln Lys Pro Gly Gln Pro Pro
        35                  40                  45
Lys Leu Leu Ile Lys Tyr Ala Ser Asn Leu Glu Ser Gly Val Pro Ala
    50                  55                  60
Arg Phe Ser Gly Ser Gly Ser Gly Thr Asp Phe Thr Leu Thr Ile Asn
65                  70                  75                  80
Pro Val Glu Ala Asn Asp Thr Ala Asn Tyr Tyr Cys Gln His Ser Trp
                85                  90                  95
Glu Ile Pro Tyr Thr Phe Gly Gln Gly Thr Lys Leu Glu Ile Lys Arg
            100                 105                 110
Thr Val Ala Ala Pro Ser Val Phe Ile Phe Pro Pro Ser Asp Glu Gln
        115                 120                 125
Leu Lys Ser Gly Thr Ala Ser Val Val Cys Leu Leu Asn Asn Phe Tyr
    130                 135                 140
Pro Arg Glu Ala Lys Val Gln Trp Lys Val Asp Asn Ala Leu Gln Ser
145                 150                 155                 160
Gly Asn Ser Gln Glu Ser Val Thr Glu Gln Asp Ser Lys Asp Ser Thr
                165                 170                 175
Tyr Ser Leu Ser Ser Thr Leu Thr Leu Ser Lys Ala Asp Tyr Glu Lys
            180                 185                 190
His Lys Val Tyr Ala Cys Glu Val Thr His Gln Gly Leu Ser Ser Pro
        195                 200                 205
Val Thr Lys Ser Phe Asn Arg Gly Glu Cys
    210                 215
```

<210> 14
<211> 654
<212> DNA
<213> Synthetic sequence

<220>
<223> Nucleic acid sequence for encoding light chain of anti-PD-L1-TGF-β RII fusion protein

<400> 14

```
gacattgtgc tgactcagag ccccgcttca ctggcagtgt ctccagggca gcgggcaacc        60
atcacatgca gagcctcaca gagcgtctcc accagctcct ctagtttcat gcactggtac       120
cagcagaagc ccggacagcc ccctaagctg ctgatcaaat atgctagcaa cctggagtcc       180
ggcgtgccag ccaggttctc tggcagtggg tcaggaaccg actttactct gaccattaat       240
cccgtcgaag ccaacgatac agctaattac tattgtcagc attcctggga gatcccttac       300
```

```
acatttggcc aggggactaa gctggagatc aagcgtacgg tggccgcacc aagcgtcttc        360
atcttcccgc catctgatga gcagttgaaa tctggaactg cctctgttgt gtgcctgctg        420
aataacttct atcccagaga ggccaaagta cagtggaagg tggataacgc cctccaatcg        480
ggtaactccc aggagagtgt cacagagcag gacagcaagg acagcaccta cagcctcagc        540
agcaccctga cgctgagcaa agcagactac gagaaacaca aagtctacgc ctgcgaagtc        600
acccatcagg gcctgagctc gcccgtcaca aagagcttta acagaggcga gtgc              654

<210>  15
<211>  5
<212>  PRT
<213>  Mus sp.

<400>  15
Thr Tyr Gly Val His
1               5

<210>  16
<211>  16
<212>  PRT
<213>  Mus sp.

<400>  16
Val Ile Trp Arg Gly Val Thr Thr Asp Tyr Asn Ala Ala Phe Met Ser
1               5                   10                  15

<210>  17
<211>  8
<212>  PRT
<213>  Mus sp.

<400>  17
Leu Gly Phe Tyr Ala Met Asp Tyr
1               5

<210>  18
<211>  11
<212>  PRT
<213>  Mus sp.

<400>  18
Lys Ala Ser Gln Ser Val Ser Asn Asp Val Ala
1               5                   10

<210>  19
<211>  7
<212>  PRT
<213>  Mus sp.

<400>  19
Tyr Ala Ala Asn Arg Tyr Thr
1               5

<210>  20
<211>  9
<212>  PRT
<213>  Mus sp.

<400>  20
```

Gln Gln Asp Tyr Thr Ser Pro Tyr Thr
1 5

<210> 21
<211> 116
<212> PRT
<213> Synthetic sequence

<220>
<223> Heavy chain variable domain of PD-L1 antibody

<400> 21
Gln Ile Thr Leu Lys Glu Ser Gly Pro Thr Leu Val Lys Pro Thr Gln
1 5 10 15
Thr Leu Thr Leu Thr Cys Thr Val Ser Gly Phe Ser Leu Ser Thr Tyr
20 25 30
Gly Val His Trp Ile Arg Gln Pro Pro Gly Lys Ala Leu Glu Trp Leu
35 40 45
Gly Val Ile Trp Arg Gly Val Thr Thr Asp Tyr Asn Ala Ala Phe Met
50 55 60
Ser Arg Leu Thr Ile Thr Lys Asp Asn Ser Lys Asn Gln Val Val Leu
65 70 75 80
Thr Met Asn Asn Met Asp Pro Val Asp Thr Ala Thr Tyr Tyr Cys Ala
85 90 95
Arg Leu Gly Phe Tyr Ala Met Asp Tyr Trp Gly Gln Gly Thr Leu Val
100 105 110
Thr Val Ser Ser
115

<210> 22
<211> 107
<212> PRT
<213> Synthetic sequence

<220>
<223> Light chain variable domain of PD-L1 antibody

<400> 22
Asp Ile Gln Met Thr Gln Ser Pro Ser Ser Leu Ser Ala Ser Val Gly
1 5 10 15
Asp Arg Val Thr Ile Thr Cys Lys Ala Ser Gln Ser Val Ser Asn Asp
20 25 30
Val Ala Trp Tyr Gln Gln Lys Pro Gly Lys Ala Pro Lys Leu Leu Ile
35 40 45
Tyr Tyr Ala Ala Asn Arg Tyr Thr Gly Val Pro Asp Arg Phe Ser Gly
50 55 60
Ser Gly Tyr Gly Thr Asp Phe Thr Phe Thr Ile Ser Ser Leu Gln Pro
65 70 75 80
Glu Asp Ile Ala Thr Tyr Phe Cys Gln Gln Asp Tyr Thr Ser Pro Tyr
85 90 95
Thr Phe Gly Gln Gly Thr Lys Leu Glu Ile Lys
100 105

<210> 23
<211> 446
<212> PRT
<213> Synthetic sequence

<220>
<223> Heavy chain of PD-L1 antibody

<400> 23

```
Gln Ile Thr Leu Lys Glu Ser Gly Pro Thr Leu Val Lys Pro Thr Gln
1               5                   10                  15
Thr Leu Thr Leu Thr Cys Thr Val Ser Gly Phe Ser Leu Ser Thr Tyr
            20                  25                  30
Gly Val His Trp Ile Arg Gln Pro Pro Gly Lys Ala Leu Glu Trp Leu
        35                  40                  45
Gly Val Ile Trp Arg Gly Val Thr Thr Asp Tyr Asn Ala Ala Phe Met
    50                  55                  60
Ser Arg Leu Thr Ile Thr Lys Asp Asn Ser Lys Asn Gln Val Val Leu
65                  70                  75                  80
Thr Met Asn Asn Met Asp Pro Val Asp Thr Ala Thr Tyr Tyr Cys Ala
                85                  90                  95
Arg Leu Gly Phe Tyr Ala Met Asp Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Ala Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
```

```
Asn His Tyr Thr Gln Lys Ser Leu Ser Leu Ser Pro Gly Lys
        435                 440                 445
```

<210> 24
<211> 214
<212> PRT
<213> Synthetic sequence

<220>
<223> Light chain of PD-L1 antibody

<400> 24

```
Asp Ile Gln Met Thr Gln Ser Pro Ser Ser Leu Ser Ala Ser Val Gly
1               5                   10                  15
Asp Arg Val Thr Ile Thr Cys Lys Ala Ser Gln Ser Val Ser Asn Asp
            20                  25                  30
Val Ala Trp Tyr Gln Gln Lys Pro Gly Lys Ala Pro Lys Leu Leu Ile
        35                  40                  45
Tyr Tyr Ala Ala Asn Arg Tyr Thr Gly Val Pro Asp Arg Phe Ser Gly
    50                  55                  60
Ser Gly Tyr Gly Thr Asp Phe Thr Phe Thr Ile Ser Ser Leu Gln Pro
65                  70                  75                  80
Glu Asp Ile Ala Thr Tyr Phe Cys Gln Gln Asp Tyr Thr Ser Pro Tyr
                85                  90                  95
Thr Phe Gly Gln Gly Thr Lys Leu Glu Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 25
<211> 603
<212> PRT
<213> Synthetic sequence

<220>
<223> Heavy chain of anti-PD-L1-TGF-β RII fusion protein

<400> 25

```
Gln Ile Thr Leu Lys Glu Ser Gly Pro Thr Leu Val Lys Pro Thr Gln
1               5                   10                  15
Thr Leu Thr Leu Thr Cys Thr Val Ser Gly Phe Ser Leu Ser Thr Tyr
            20                  25                  30
Gly Val His Trp Ile Arg Gln Pro Pro Gly Lys Ala Leu Glu Trp Leu
        35                  40                  45
Gly Val Ile Trp Arg Gly Val Thr Thr Asp Tyr Asn Ala Ala Phe Met
    50                  55                  60
```

```
Ser Arg Leu Thr Ile Thr Lys Asp Asn Ser Lys Asn Gln Val Val Leu
65                  70                  75                  80
Thr Met Asn Asn Met Asp Pro Val Asp Thr Ala Thr Tyr Tyr Cys Ala
                85                  90                  95
Arg Leu Gly Phe Tyr Ala Met Asp Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Ala Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys Gly Gly
        435                 440                 445
Gly Gly Ser Gly Gly Gly Gly Ser Gly Gly Gly Gly Ser Gly Gly Gly
    450                 455                 460
Gly Ser Gly Ile Pro Pro His Val Gln Lys Ser Val Asn Asn Asp Met
465                 470                 475                 480
Ile Val Thr Asp Asn Asn Gly Ala Val Lys Phe Pro Gln Leu Cys Lys
                485                 490                 495
Phe Cys Asp Val Arg Phe Ser Thr Cys Asp Asn Gln Lys Ser Cys Met
            500                 505                 510
Ser Asn Cys Ser Ile Thr Ser Ile Cys Glu Lys Pro Gln Glu Val Cys
        515                 520                 525
```

```
Val Ala Val Trp Arg Lys Asn Asp Glu Asn Ile Thr Leu Glu Thr Val
    530                 535                 540
Cys His Asp Pro Lys Leu Pro Tyr His Asp Phe Ile Leu Glu Asp Ala
545                 550                 555                 560
Ala Ser Pro Lys Cys Ile Met Lys Glu Lys Lys Lys Pro Gly Glu Thr
                565                 570                 575
Phe Phe Met Cys Ser Cys Ser Ser Asp Glu Cys Asn Asp Asn Ile Ile
            580                 585                 590
Phe Ser Glu Glu Tyr Asn Thr Ser Asn Pro Asp
        595                 600
```

```
<210>  26
<211>  1809
<212>  DNA
<213>  Synthetic sequence

<220>
<223>  Nucleic acid sequence for encoding heavy chain of anti-PD-L1-TGF-β RII
fusion protein

<400>  26
cagatcacac tgaaagaaag cggccctacc ctggtcaagc caactcagac cctgacactg      60
acttgcaccg tgtctgggtt ctctctgagt acatacggag tccactggat caggcagccc     120
cctggcaaag ctctggagtg gctgggagtg atttggcggg gcgtcaccac agactataac     180
gccgctttta tgtcaagact gacaatcact aaggataaca gcaaaaatca ggtggtcctg     240
accatgaaca atatggaccc cgtggatacc gcaacatact attgtgcccg gctggggttc     300
tacgccatgg actattgggg ccaggggact ctggtgaccg tctcgagcgc ctccactaag     360
ggaccatccg tgttccctct ggcaccctcc agcaagagca caagcggagg caccgccgca     420
ctgggctgcc tcgtgaagga ctacttccca gaacccgtga ccgtcagctg gaatagcggc     480
gctctgacca gcggagtcca cactttcccc gcagtgctgc agtccagcgg cctgtacagc     540
ctgagcagcg tggtcactgt gccaagcagc agcctgggca ctcagaccta catctgcaac     600
gtcaaccaca agcccagcaa cacaaaggtg gacaagaagg tcgagcccaa gtcctgcgat     660
aagacccaca cctgccctcc atgtcccgcc cccgagctgc tgggaggacc cagcgtcttc     720
ctgtttcccc ccaagccaaa ggacaccctg atgatcagca ggacccccga agtgacctgc     780
gtcgtggtgg ccgtgagcca cgaagatccc gaggtgaagt tcaactggta cgtggacggc     840
gtggaagtgc acaacgccaa gacaaaaccc agggaggagc agtataacag cacctacagg     900
gtcgtgagcg tcctgaccgt gctgcaccaa gactggctga acggcaagga gtataagtgc     960
aaggtgagca acaaggcact gcccgccccc atcgagaaga ccatttccaa ggccaagggg    1020
caacctaggg agccacaggt ctacactctg ccccctagca gggacgagct gaccaagaac    1080
caggtctccc tgacttgcct ggtgaagggg ttttatccca gcgacatcgc cgtcgagtgg    1140
gagagcaatg gccagcccga aaacaactac aagaccacac cccctgtgct ggacagcgac    1200
ggcagcttct ttctgtatag caaactgaca gtggataaga gcagatggca gcagggcaac    1260
gtgttctcct gctccgtgat gcacgaggcc ctgcacaatc actacaccca gaagtccctg    1320
agcctgtccc ccggaaaagg aggaggagga tctggaggag gcggcagcgg cggaggagga    1380
agtggaggag gcggatccgg catccctccc cacgtgcaga agagcgtgaa taacgacatg    1440
atcgtgaccg acaataacgg cgctgtgaag ttccctcagc tgtgcaagtt ctgcgatgtg    1500
cggttctcca cctgcgacaa tcagaagagc tgcatgagca actgcagcat cacctccatc    1560
tgcgagaagc ctcaggaggt gtgtgtggcc gtgtggcgga agaatgacga gaatatcacc    1620
ctggagaccg tgtgccacga ccccaagctg ccttatcacg atttcatcct ggaggacgcc    1680
gctagcccca agtgcatcat gaaggagaag aagaagcccg gcgagacctt cttcatgtgt    1740
agctgtagca gcgatgagtg caacgataat atcatcttta gcgaggagta taacaccagc    1800
aatcccgat                                                            1809

<210>  27
<211>  214
<212>  PRT
<213>  Synthetic sequence
```

<220>
<223> Light chain of anti-PD-L1-TGF-β RII fusion protein

<400> 27
Asp Ile Gln Met Thr Gln Ser Pro Ser Ser Leu Ser Ala Ser Val Gly
1 5 10 15
Asp Arg Val Thr Ile Thr Cys Lys Ala Ser Gln Ser Val Ser Asn Asp
20 25 30
Val Ala Trp Tyr Gln Gln Lys Pro Gly Lys Ala Pro Lys Leu Leu Ile
35 40 45
Tyr Tyr Ala Ala Asn Arg Tyr Thr Gly Val Pro Asp Arg Phe Ser Gly
50 55 60
Ser Gly Tyr Gly Thr Asp Phe Thr Phe Thr Ile Ser Ser Leu Gln Pro
65 70 75 80
Glu Asp Ile Ala Thr Tyr Phe Cys Gln Gln Asp Tyr Thr Ser Pro Tyr
85 90 95
Thr Phe Gly Gln Gly Thr Lys Leu Glu Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 28
<211> 642
<212> DNA
<213> Synthetic sequence

<220>
<223> Nucleic acid sequence for encoding light chain of anti-PD-L1-TGF-β RII fusion protein

<400> 28
gacatccaga tgactcagtc tccaagcagc ctgtctgcat ctgtggggga cagggtcacc 60
atcacatgca aagcatctca gagtgtgtca aacgatgtcg cctggtacca gcagaagccc 120
ggaaaagctc ctaagctgct gatttactat gccgctaatc ggtacactgg cgtgccagac 180
agattcagcg gatccggata tggaaccgat ttcactttta ccatcagctc cctgcagcca 240
gaggacattg ccacatattt ctgtcagcag gattacacaa gcccctatac ttttggccag 300
gggaccaaac tggaaatcaa gcgtacggtg gccgcaccaa gcgtcttcat cttcccgcca 360
tctgatgagc agttgaaatc tggaactgcc tctgttgtgt gcctgctgaa taacttctat 420
cccagagagg ccaaagtaca gtggaaggtg gataacgccc tccaatcggg taactcccag 480
gagagtgtca cagagcagga cagcaaggac agcacctaca gcctcagcag caccctgacg 540
ctgagcaaag cagactacga gaaacacaaa gtctacgcct gcgaagtcac ccatcagggc 600
ctgagctcgc ccgtcacaaa gagctttaac agaggcgagt gc 642

<210> 29
<211> 446
<212> PRT

<213> Synthetic sequence

<220>
<223> Heavy chain of PD-L1 antibody

<400> 29

```
Glu Val Gln Leu Val Glu Ser Gly Gly Gly Leu Val Lys Pro Gly Gly
1               5                   10                  15
Ser Leu Arg Leu Ser Cys Ala Ala Ser Gly Phe Ile Phe Arg Ser Tyr
            20                  25                  30
Gly Met Ser Trp Val Arg Gln Ala Pro Gly Lys Gly Leu Glu Trp Val
        35                  40                  45
Ala Ser Ile Ser Ser Gly Gly Ser Thr Tyr Tyr Pro Asp Ser Val Lys
    50                  55                  60
Gly Arg Phe Thr Ile Ser Arg Asp Asn Ala Lys Asn Ser Leu Tyr Leu
65                  70                  75                  80
Gln Met Asn Ser Leu Arg Ala Glu Asp Thr Ala Val Tyr Asp Cys Ala
                85                  90                  95
Arg Gly Tyr Asp Ser Gly Phe Ala Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Ala Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
```

Gln Gln Gly Asn Val Phe Ser Cys Ser Val Met His Glu Ala Leu His
420 425 430
Asn His Tyr Thr Gln Lys Ser Leu Ser Leu Ser Pro Gly Ala
435 440 445

<210> 30
<211> 603
<212> PRT
<213> Synthetic sequence

<220>
<223> Heavy chain of anti-PD-L1-TGF-β RII fusion protein

<400> 30
Glu Val Gln Leu Val Glu Ser Gly Gly Gly Leu Val Lys Pro Gly Gly
1 5 10 15
Ser Leu Arg Leu Ser Cys Ala Ala Ser Gly Phe Ile Phe Arg Ser Tyr
20 25 30
Gly Met Ser Trp Val Arg Gln Ala Pro Gly Lys Gly Leu Glu Trp Val
35 40 45
Ala Ser Ile Ser Ser Gly Gly Ser Thr Tyr Tyr Pro Asp Ser Val Lys
50 55 60
Gly Arg Phe Thr Ile Ser Arg Asp Asn Ala Lys Asn Ser Leu Tyr Leu
65 70 75 80
Gln Met Asn Ser Leu Arg Ala Glu Asp Thr Ala Val Tyr Asp Cys Ala
85 90 95
Arg Gly Tyr Asp Ser Gly Phe Ala Tyr Trp Gly Gln Gly Thr Leu Val
100 105 110
Thr Val Ser Ser Ala Ser Thr Lys Gly Pro Ser Val Phe Pro Leu Ala
115 120 125
Pro Ser Ser Lys Ser Thr Ser Gly Gly Thr Ala Ala Leu Gly Cys Leu
130 135 140
Val Lys Asp Tyr Phe Pro Glu Pro Val Thr Val Ser Trp Asn Ser Gly
145 150 155 160
Ala Leu Thr Ser Gly Val His Thr Phe Pro Ala Val Leu Gln Ser Ser
165 170 175
Gly Leu Tyr Ser Leu Ser Ser Val Val Thr Val Pro Ser Ser Ser Leu
180 185 190
Gly Thr Gln Thr Tyr Ile Cys Asn Val Asn His Lys Pro Ser Asn Thr
195 200 205
Lys Val Asp Lys Lys Val Glu Pro Lys Ser Cys Asp Lys Thr His Thr
210 215 220
Cys Pro Pro Cys Pro Ala Pro Glu Leu Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Ala Val Ser His Glu Asp Pro Glu Val
260 265 270
Lys Phe Asn Trp Tyr Val Asp Gly Val Glu Val His Asn Ala Lys Thr
275 280 285
Lys Pro Arg Glu Glu Gln Tyr Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Ala Leu Pro Ala Pro Ile Glu Lys Thr Ile Ser
325 330 335
Lys Ala Lys Gly Gln Pro Arg Glu Pro Gln Val Tyr Thr Leu Pro Pro
340 345 350

```
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Ala Gly Gly
        435                 440                 445
Gly Gly Ser Gly Gly Gly Gly Ser Gly Gly Gly Gly Ser Gly Gly Gly
    450                 455                 460
Gly Ser Gly Ile Pro Pro His Val Gln Lys Ser Val Asn Asn Asp Met
465                 470                 475                 480
Ile Val Thr Asp Asn Asn Gly Ala Val Lys Phe Pro Gln Leu Cys Lys
                485                 490                 495
Phe Cys Asp Val Arg Phe Ser Thr Cys Asp Asn Gln Lys Ser Cys Met
            500                 505                 510
Ser Asn Cys Ser Ile Thr Ser Ile Cys Glu Lys Pro Gln Glu Val Cys
        515                 520                 525
Val Ala Val Trp Arg Lys Asn Asp Glu Asn Ile Thr Leu Glu Thr Val
    530                 535                 540
Cys His Asp Pro Lys Leu Pro Tyr His Asp Phe Ile Leu Glu Asp Ala
545                 550                 555                 560
Ala Ser Pro Lys Cys Ile Met Lys Glu Lys Lys Lys Pro Gly Glu Thr
                565                 570                 575
Phe Phe Met Cys Ser Cys Ser Ser Asp Glu Cys Asn Asp Asn Ile Ile
            580                 585                 590
Phe Ser Glu Glu Tyr Asn Thr Ser Asn Pro Asp
        595                 600
```

<210> 31
<211> 1809
<212> DNA
<213> Synthetic sequence

<220>
<223> Nucleic acid sequence for encoding heavy chain of anti-PD-L1-TGF-β RII fusion protein

<400> 31

```
gaggtgcagc tggtcgagtc aggagggggg ctggtcaagc caggagggtc actgcgactg      60
agctgcgcag cttccgggtt catctttagg tcttatggca tgagttgggt gcgccaggca     120
ccagggaaag gactggagtg ggtcgcttca atcagctccg gaggcagcac ttactatcct     180
gactccgtga agggccggtt caccatttct agagataacg ccaaaaatag tctgtacctg     240
cagatgaact ctctgcgagc agaagacaca gccgtctacg attgtgctag aggatatgac     300
agcggctttg catactgggg ccaggggacc ctggtgacag tctcgagcgc ctccactaag     360
ggcccatccg tgttccctct ggcaccctcc agcaagagca caagcggagg caccgccgca     420
ctgggctgcc tcgtgaagga ctacttccca gaacccgtga ccgtcagctg gaatagcggc     480
gctctgacca gcggagtcca cactttcccc gcagtgctgc agtccagcgg cctgtacagc     540
ctgagcagcg tggtcactgt gccaagcagc agcctgggca ctcagaccta catctgcaac     600
gtcaaccaca agcccagcaa cacaaaggtg gacaagaagg tcgagcccaa gtcctgcgat     660
aagacccaca cctgccctcc atgtcccgcc cccgagctgc tgggaggacc cagcgtcttc     720
ctgtttcccc ccaagccaaa ggacaccctg atgatcagca ggacccccga agtgacctgc     780
gtcgtggtgg ccgtgagcca cgaagatccc gaggtgaagt tcaactggta cgtggacggc     840
gtggaagtgc acaacgccaa gacaaaaccc agggaggagc agtataacag cacctacagg     900
```

```
gtcgtgagcg tcctgaccgt gctgcaccaa gactggctga acggcaagga gtataagtgc        960
aaggtgagca acaaggcact gcccgccccc atcgagaaga ccatttccaa ggccaagggg       1020
caacctaggg agccacaggt ctacactctg cccccctagca gggacgagct gaccaagaac      1080
caggtctccc tgacttgcct ggtgaagggg ttttatccca gcgacatcgc cgtcgagtgg       1140
gagagcaatg gccagcccga aaacaactac aagaccacac cccctgtgct ggacagcgac       1200
ggcagcttct ttctgtatag caaactgaca gtggataaga gcagatggca gcagggcaac       1260
gtgttctcct gctccgtgat gcacgaggcc ctgcacaatc actacaccca gaagtccctg       1320
agcctgtccc ccggagccgg aggaggagga tctggaggag gcggcagcgg cggaggagga       1380
agtggaggag gcggatccgg catccctccc cacgtgcaga agagcgtgaa taacgacatg       1440
atcgtgaccg acaataacgg cgctgtgaag ttccctcagc tgtgcaagtt ctgcgatgtg       1500
cggttctcca cctgcgacaa tcagaagagc tgcatgagca actgcagcat cacctccatc       1560
tgcgagaagc ctcaggaggt gtgtgtggcc gtgtggcgga agaatgacga gaatatcacc       1620
ctggagaccg tgtgccacga ccccaagctg ccttatcacg atttcatcct ggaggacgcc       1680
gctagcccca agtgcatcat gaaggagaag aagaagcccg gcgagacctt cttcatgtgt       1740
agctgtagca gcgatgagtg caacgataat atcatcttta gcgaggagta taacaccagc       1800
aatcccgat                                                               1809
```

<210> 32
<211> 446
<212> PRT
<213> Synthetic sequence

<220>
<223> Heavy chain of PD-L1 antibody

<400> 32

```
Gln Ile Thr Leu Lys Glu Ser Gly Pro Thr Leu Val Lys Pro Thr Gln
1               5                   10                  15
Thr Leu Thr Leu Thr Cys Thr Val Ser Gly Phe Ser Leu Ser Thr Tyr
            20                  25                  30
Gly Val His Trp Ile Arg Gln Pro Pro Gly Lys Ala Leu Glu Trp Leu
        35                  40                  45
Gly Val Ile Trp Arg Gly Val Thr Thr Asp Tyr Asn Ala Ala Phe Met
    50                  55                  60
Ser Arg Leu Thr Ile Thr Lys Asp Asn Ser Lys Asn Gln Val Val Leu
65                  70                  75                  80
Thr Met Asn Asn Met Asp Pro Val Asp Thr Ala Thr Tyr Tyr Cys Ala
                85                  90                  95
Arg Leu Gly Phe Tyr Ala Met Asp Tyr Trp Gly Gln Gly Thr Leu Val
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
```

Glu Val Thr Cys Val Val Val Ala Val Ser His Glu Asp Pro Glu Val
260 265 270
Lys Phe Asn Trp Tyr Val Asp Gly Val Glu Val His Asn Ala Lys Thr
275 280 285
Lys Pro Arg Glu Glu Gln Tyr Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Ala Leu Pro Ala Pro Ile Glu Lys Thr Ile Ser
325 330 335
Lys Ala Lys Gly Gln Pro Arg Glu Pro Gln Val Tyr Thr Leu Pro Pro
340 345 350
Ser Arg Asp Glu Leu Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Lys Leu Thr Val Asp Lys Ser Arg Trp
405 410 415
Gln Gln Gly Asn Val Phe Ser Cys Ser Val Met His Glu Ala Leu His
420 425 430
Asn His Tyr Thr Gln Lys Ser Leu Ser Leu Ser Pro Gly Ala
435 440 445

<210> 33
<211> 603
<212> PRT
<213> Synthetic sequence

<220>
<223> Heavy chain of anti-PD-L1-TGF-β RII fusion protein

<400> 33
Gln Ile Thr Leu Lys Glu Ser Gly Pro Thr Leu Val Lys Pro Thr Gln
1 5 10 15
Thr Leu Thr Leu Thr Cys Thr Val Ser Gly Phe Ser Leu Ser Thr Tyr
20 25 30
Gly Val His Trp Ile Arg Gln Pro Pro Gly Lys Ala Leu Glu Trp Leu
35 40 45
Gly Val Ile Trp Arg Gly Val Thr Thr Asp Tyr Asn Ala Ala Phe Met
50 55 60
Ser Arg Leu Thr Ile Thr Lys Asp Asn Ser Lys Asn Gln Val Val Leu
65 70 75 80
Thr Met Asn Asn Met Asp Pro Val Asp Thr Ala Thr Tyr Tyr Cys Ala
85 90 95
Arg Leu Gly Phe Tyr Ala Met Asp Tyr Trp Gly Gln Gly Thr Leu Val
100 105 110
Thr Val Ser Ser Ala Ser Thr Lys Gly Pro Ser Val Phe Pro Leu Ala
115 120 125
Pro Ser Ser Lys Ser Thr Ser Gly Gly Thr Ala Ala Leu Gly Cys Leu
130 135 140
Val Lys Asp Tyr Phe Pro Glu Pro Val Thr Val Ser Trp Asn Ser Gly
145 150 155 160
Ala Leu Thr Ser Gly Val His Thr Phe Pro Ala Val Leu Gln Ser Ser
165 170 175
Gly Leu Tyr Ser Leu Ser Ser Val Val Thr Val Pro Ser Ser Ser Leu
180 185 190

```
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Ala Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Ala Gly Gly
        435                 440                 445
Gly Gly Ser Gly Gly Gly Gly Ser Gly Gly Gly Gly Ser Gly Gly Gly
    450                 455                 460
Gly Ser Gly Ile Pro Pro His Val Gln Lys Ser Val Asn Asn Asp Met
465                 470                 475                 480
Ile Val Thr Asp Asn Asn Gly Ala Val Lys Phe Pro Gln Leu Cys Lys
                485                 490                 495
Phe Cys Asp Val Arg Phe Ser Thr Cys Asp Asn Gln Lys Ser Cys Met
            500                 505                 510
Ser Asn Cys Ser Ile Thr Ser Ile Cys Glu Lys Pro Gln Glu Val Cys
        515                 520                 525
Val Ala Val Trp Arg Lys Asn Asp Glu Asn Ile Thr Leu Glu Thr Val
    530                 535                 540
Cys His Asp Pro Lys Leu Pro Tyr His Asp Phe Ile Leu Glu Asp Ala
545                 550                 555                 560
Ala Ser Pro Lys Cys Ile Met Lys Glu Lys Lys Lys Pro Gly Glu Thr
                565                 570                 575
Phe Phe Met Cys Ser Cys Ser Ser Asp Glu Cys Asn Asp Asn Ile Ile
            580                 585                 590
Phe Ser Glu Glu Tyr Asn Thr Ser Asn Pro Asp
        595                 600
```

<210> 34
<211> 1809
<212> DNA
<213> Synthetic sequence

<220>
<223> Nucleic acid sequence for encoding heavy chain of anti-PD-L1-TGF-β RII fusion protein

<400> 34
```
cagatcacac tgaaagaaag cggccctacc ctggtcaagc caactcagac cctgacactg      60
acttgcaccg tgtctgggtt ctctctgagt acatacggag tccactggat caggcagccc     120
cctggcaaag ctctggagtg gctgggagtg atttggcggg gcgtcaccac agactataac     180
gccgctttta tgtcaagact gacaatcact aaggataaca gcaaaaatca ggtggtcctg     240
accatgaaca atatggaccc cgtggatacc gcaacatact attgtgcccg gctggggttc     300
tacgccatgg actattgggg ccaggggact ctggtgaccg tctcgagcgc ctccactaag     360
ggaccatccg tgttccctct ggcaccctcc agcaagagca caagcggagg caccgccgca     420
ctgggctgcc tcgtgaagga ctacttccca gaacccgtga ccgtcagctg gaatagcggc     480
gctctgacca gcggagtcca cactttcccc gcagtgctgc agtccagcgg cctgtacagc     540
ctgagcagcg tggtcactgt gccaagcagc agcctgggca ctcagaccta catctgcaac     600
gtcaaccaca agcccagcaa cacaaaggtg gacaagaagg tcgagcccaa gtcctgcgat     660
aagacccaca cctgccctcc atgtcccgcc cccgagctgc tgggaggacc cagcgtcttc     720
ctgtttcccc ccaagccaaa ggacaccctg atgatcagca ggacccccga agtgacctgc     780
gtcgtggtgg ccgtgagcca cgaagatccc gaggtgaagt tcaactggta cgtggacggc     840
gtggaagtgc acaacgccaa gacaaaaccc agggaggagc agtataacag cacctacagg     900
gtcgtgagcg tcctgaccgt gctgcaccaa gactggctga acggcaagga gtataagtgc     960
aaggtgagca acaaggcact gcccgccccc atcgagaaga ccatttccaa ggccaagggg    1020
caacctaggg agccacaggt ctacactctg cccccctagca gggacgagct gaccaagaac    1080
caggtctccc tgacttgcct ggtgaagggg ttttatccca gcgacatcgc cgtcgagtgg    1140
gagagcaatg gccagcccga aaacaactac aagaccacac cccctgtgct ggacagcgac    1200
ggcagcttct ttctgtatag caaactgaca gtggataaga gcagatggca gcagggcaac    1260
gtgttctcct gctccgtgat gcacgaggcc ctgcacaatc actacaccca gaagtccctg    1320
agcctgtccc ccggagccgg aggaggagga tctggaggag gcggcagcgg cggaggagga    1380
agtggaggag gcggatccgg catccctccc cacgtgcaga agagcgtgaa taacgacatg    1440
atcgtgaccg acaataacgg cgctgtgaag ttccctcagc tgtgcaagtt ctgcgatgtg    1500
cggttctcca cctgcgacaa tcagaagagc tgcatgagca actgcagcat cacctccatc    1560
tgcgagaagc ctcaggaggt gtgtgtggcc gtgtggcgga agaatgacga gaatatcacc    1620
ctggagaccg tgtgccacga ccccaagctg ccttatcacg atttcatcct ggaggacgcc    1680
gctagcccca agtgcatcat gaaggagaag aagaagcccg gcgagacctt cttcatgtgt    1740
agctgtagca gcgatgagtg caacgataat atcatcttta gcgaggagta taacaccagc    1800
aatcccgat                                                            1809
```

<210> 35
<211> 567
<212> PRT
<213> Synthetic sequence

<220>
<223> Human TGFβ RII isoform B precursor polypeptide

<400> 35
```
Met Gly Arg Gly Leu Leu Arg Gly Leu Trp Pro Leu His Ile Val Leu
1               5                   10                  15
Trp Thr Arg Ile Ala Ser Thr Ile Pro Pro His Val Gln Lys Ser Val
            20                  25                  30
Asn Asn Asp Met Ile Val Thr Asp Asn Asn Gly Ala Val Lys Phe Pro
        35                  40                  45
Gln Leu Cys Lys Phe Cys Asp Val Arg Phe Ser Thr Cys Asp Asn Gln
    50                  55                  60
Lys Ser Cys Met Ser Asn Cys Ser Ile Thr Ser Ile Cys Glu Lys Pro
65                  70                  75                  80
Gln Glu Val Cys Val Ala Val Trp Arg Lys Asn Asp Glu Asn Ile Thr
                85                  90                  95
```

```
Leu Glu Thr Val Cys His Asp Pro Lys Leu Pro Tyr His Asp Phe Ile
            100                 105                 110
Leu Glu Asp Ala Ala Ser Pro Lys Cys Ile Met Lys Glu Lys Lys Lys
        115                 120                 125
Pro Gly Glu Thr Phe Phe Met Cys Ser Cys Ser Ser Asp Glu Cys Asn
    130                 135                 140
Asp Asn Ile Ile Phe Ser Glu Glu Tyr Asn Thr Ser Asn Pro Asp Leu
145                 150                 155                 160
Leu Leu Val Ile Phe Gln Val Thr Gly Ile Ser Leu Leu Pro Pro Leu
                165                 170                 175
Gly Val Ala Ile Ser Val Ile Ile Ile Phe Tyr Cys Tyr Arg Val Asn
            180                 185                 190
Arg Gln Gln Lys Leu Ser Ser Thr Trp Glu Thr Gly Lys Thr Arg Lys
        195                 200                 205
Leu Met Glu Phe Ser Glu His Cys Ala Ile Ile Leu Glu Asp Asp Arg
    210                 215                 220
Ser Asp Ile Ser Ser Thr Cys Ala Asn Asn Ile Asn His Asn Thr Glu
225                 230                 235                 240
Leu Leu Pro Ile Glu Leu Asp Thr Leu Val Gly Lys Gly Arg Phe Ala
                245                 250                 255
Glu Val Tyr Lys Ala Lys Leu Lys Gln Asn Thr Ser Glu Gln Phe Glu
            260                 265                 270
Thr Val Ala Val Lys Ile Phe Pro Tyr Glu Glu Tyr Ala Ser Trp Lys
        275                 280                 285
Thr Glu Lys Asp Ile Phe Ser Asp Ile Asn Leu Lys His Glu Asn Ile
    290                 295                 300
Leu Gln Phe Leu Thr Ala Glu Glu Arg Lys Thr Glu Leu Gly Lys Gln
305                 310                 315                 320
Tyr Trp Leu Ile Thr Ala Phe His Ala Lys Gly Asn Leu Gln Glu Tyr
                325                 330                 335
Leu Thr Arg His Val Ile Ser Trp Glu Asp Leu Arg Lys Leu Gly Ser
            340                 345                 350
Ser Leu Ala Arg Gly Ile Ala His Leu His Ser Asp His Thr Pro Cys
        355                 360                 365
Gly Arg Pro Lys Met Pro Ile Val His Arg Asp Leu Lys Ser Ser Asn
    370                 375                 380
Ile Leu Val Lys Asn Asp Leu Thr Cys Cys Leu Cys Asp Phe Gly Leu
385                 390                 395                 400
Ser Leu Arg Leu Asp Pro Thr Leu Ser Val Asp Asp Leu Ala Asn Ser
                405                 410                 415
Gly Gln Val Gly Thr Ala Arg Tyr Met Ala Pro Glu Val Leu Glu Ser
            420                 425                 430
Arg Met Asn Leu Glu Asn Val Glu Ser Phe Lys Gln Thr Asp Val Tyr
        435                 440                 445
Ser Met Ala Leu Val Leu Trp Glu Met Thr Ser Arg Cys Asn Ala Val
    450                 455                 460
Gly Glu Val Lys Asp Tyr Glu Pro Pro Phe Gly Ser Lys Val Arg Glu
465                 470                 475                 480
His Pro Cys Val Glu Ser Met Lys Asp Asn Val Leu Arg Asp Arg Gly
                485                 490                 495
Arg Pro Glu Ile Pro Ser Phe Trp Leu Asn His Gln Gly Ile Gln Met
            500                 505                 510
Val Cys Glu Thr Leu Thr Glu Cys Trp Asp His Asp Pro Glu Ala Arg
        515                 520                 525
Leu Thr Ala Gln Cys Val Ala Glu Arg Phe Ser Glu Leu Glu His Leu
    530                 535                 540
Asp Arg Leu Ser Gly Arg Ser Cys Ser Glu Glu Lys Ile Pro Glu Asp
545                 550                 555                 560
```

Gly Ser Leu Asn Thr Thr Lys
565

<210> 36
<211> 136
<212> PRT
<213> Synthetic sequence

<220>
<223> Human TGFβ RII extracellular domain polypeptide

<400> 36
Ile Pro Pro His Val Gln Lys Ser Val Asn Asn Asp Met Ile Val Thr
1 5 10 15
Asp Asn Asn Gly Ala Val Lys Phe Pro Gln Leu Cys Lys Phe Cys Asp
20 25 30
Val Arg Phe Ser Thr Cys Asp Asn Gln Lys Ser Cys Met Ser Asn Cys
35 40 45
Ser Ile Thr Ser Ile Cys Glu Lys Pro Gln Glu Val Cys Val Ala Val
50 55 60
Trp Arg Lys Asn Asp Glu Asn Ile Thr Leu Glu Thr Val Cys His Asp
65 70 75 80
Pro Lys Leu Pro Tyr His Asp Phe Ile Leu Glu Asp Ala Ala Ser Pro
85 90 95
Lys Cys Ile Met Lys Glu Lys Lys Lys Pro Gly Glu Thr Phe Phe Met
100 105 110
Cys Ser Cys Ser Ser Asp Glu Cys Asn Asp Asn Ile Ile Phe Ser Glu
115 120 125
Glu Tyr Asn Thr Ser Asn Pro Asp
130 135

<210> 37
<211> 21
<212> PRT
<213> Synthetic sequence

<220>
<223> Linker peptide

<400> 37
Gly Gly Gly Gly Ser Gly Gly Gly Gly Ser Gly Gly Gly Gly Ser Gly
1 5 10 15
Gly Gly Gly Ser Gly
20

<210> 38
<211> 446
<212> PRT
<213> Synthetic sequence

<220>
<223> Heavy chain of IgG1

<400> 38
Glu Val Gln Leu Glu Gln Ser Gly Ala Glu Leu Met Lys Pro Gly Ala
1 5 10 15
Ser Val Lys Ile Ser Cys Lys Ala Thr Gly Tyr Thr Phe Thr Thr Tyr
20 25 30

```
Trp Ile Glu Trp Ile Lys Gln Arg Pro Gly His Ser Leu Glu Trp Ile
        35                  40                  45
Gly Glu Ile Leu Pro Gly Ser Asp Ser Thr Tyr Tyr Asn Glu Lys Val
    50                  55                  60
Lys Gly Lys Val Thr Phe Thr Ala Asp Ala Ser Ser Asn Thr Ala Tyr
65                  70                  75                  80
Met Gln Leu Ser Ser Leu Thr Ser Glu Asp Ser Ala Val Tyr Tyr Cys
                85                  90                  95
Ala Arg Gly Asp Gly Phe Tyr Val Tyr Trp Gly Gln Gly Thr Thr Leu
            100                 105                 110
Thr Val Ser Ser Ala Ser Thr Lys Gly Pro Ser Val Phe Pro Leu Ala
        115                 120                 125
Pro Ser Ser Lys Ser Thr Ser Gly Gly Thr Ala Ala Leu Gly Cys Leu
    130                 135                 140
Val Lys Asp Tyr Phe Pro Glu Pro Val Thr Val Ser Trp Asn Ser Gly
145                 150                 155                 160
Ala Leu Thr Ser Gly Val His Thr Phe Pro Ala Val Leu Gln Ser Ser
                165                 170                 175
Gly Leu Tyr Ser Leu Ser Ser Val Val Thr Val Pro Ser Ser Ser Leu
            180                 185                 190
Gly Thr Gln Thr Tyr Ile Cys Asn Val Asn His Lys Pro Ser Asn Thr
        195                 200                 205
Lys Val Asp Lys Lys Val Glu Pro Lys Ser Cys Asp Lys Thr His Thr
    210                 215                 220
Cys Pro Pro Cys Pro Ala Pro Glu Leu Leu Gly Gly Pro Ser Val Phe
225                 230                 235                 240
Leu Phe Pro Pro Lys Pro Lys Asp Thr Leu Met Ile Ser Arg Thr Pro
                245                 250                 255
Glu Val Thr Cys Val Val Val Ala Val Ser His Glu Asp Pro Glu Val
            260                 265                 270
Lys Phe Asn Trp Tyr Val Asp Gly Val Glu Val His Asn Ala Lys Thr
        275                 280                 285
Lys Pro Arg Glu Glu Gln Tyr Asn Ser Thr Tyr Arg Val Val Ser Val
    290                 295                 300
Leu Thr Val Leu His Gln Asp Trp Leu Asn Gly Lys Glu Tyr Lys Cys
305                 310                 315                 320
Lys Val Ser Asn Lys Ala Leu Pro Ala Pro Ile Glu Lys Thr Ile Ser
                325                 330                 335
Lys Ala Lys Gly Gln Pro Arg Glu Pro Gln Val Tyr Thr Leu Pro Pro
            340                 345                 350
Ser Arg Asp Glu Leu Thr Lys Asn Gln Val Ser Leu Thr Cys Leu Val
        355                 360                 365
Lys Gly Phe Tyr Pro Ser Asp Ile Ala Val Glu Trp Glu Ser Asn Gly
    370                 375                 380
Gln Pro Glu Asn Asn Tyr Lys Thr Thr Pro Pro Val Leu Asp Ser Asp
385                 390                 395                 400
Gly Ser Phe Phe Leu Tyr Ser Lys Leu Thr Val Asp Lys Ser Arg Trp
                405                 410                 415
Gln Gln Gly Asn Val Phe Ser Cys Ser Val Met His Glu Ala Leu His
            420                 425                 430
Asn His Tyr Thr Gln Lys Ser Leu Ser Leu Ser Pro Gly Lys
        435                 440                 445
```

<210> 39
<211> 214
<212> PRT
<213> Synthetic sequence

<220>
<223> Light chain of IgG1

<400> 39

```
Asp Ile Glu Leu Thr Gln Ser Pro Ala Thr Leu Ser Val Thr Pro Gly
1               5                   10                  15
Asp Ser Val Ser Leu Ser Cys Arg Ala Ser Gln Ser Ile Ser Asn Asn
            20                  25                  30
Leu His Trp Tyr Gln Gln Lys Ser His Glu Ser Pro Arg Leu Leu Ile
        35                  40                  45
Lys Tyr Thr Ser Gln Ser Met Ser Gly Ile Pro Ser Arg Phe Ser Gly
    50                  55                  60
Ser Gly Ser Gly Thr Asp Phe Thr Leu Ser Ile Asn Ser Val Glu Thr
65                  70                  75                  80
Glu Asp Phe Gly Val Tyr Phe Cys Gln Gln Ser Gly Ser Trp Pro Arg
                85                  90                  95
Thr Phe Gly Gly Gly Thr Lys Leu Asp Ile Lys Arg Thr Val Ala Ala
            100                 105                 110
Pro Ser Val Phe Ile Phe Pro Pro Ser Asp Glu Gln Leu Lys Ser Gly
        115                 120                 125
Thr Ala Ser Val Val Cys Leu Leu Asn Asn Phe Tyr Pro Arg Glu Ala
    130                 135                 140
Lys Val Gln Trp Lys Val Asp Asn Ala Leu Gln Ser Gly Asn Ser Gln
145                 150                 155                 160
Glu Ser Val Thr Glu Gln Asp Ser Lys Asp Ser Thr Tyr Ser Leu Ser
                165                 170                 175
Ser Thr Leu Thr Leu Ser Lys Ala Asp Tyr Glu Lys His Lys Val Tyr
            180                 185                 190
Ala Cys Glu Val Thr His Gln Gly Leu Ser Ser Pro Val Thr Lys Ser
        195                 200                 205
Phe Asn Arg Gly Glu Cys
    210
```

<210> 40
<211> 603
<212> PRT
<213> Synthetic sequence

<220>
<223> Heavy chain of IgG1-TGFβ RII

<400> 40

```
Glu Val Gln Leu Glu Gln Ser Gly Ala Glu Leu Met Lys Pro Gly Ala
1               5                   10                  15
Ser Val Lys Ile Ser Cys Lys Ala Thr Gly Tyr Thr Phe Thr Thr Tyr
            20                  25                  30
Trp Ile Glu Trp Ile Lys Gln Arg Pro Gly His Ser Leu Glu Trp Ile
        35                  40                  45
Gly Glu Ile Leu Pro Gly Ser Asp Ser Thr Tyr Tyr Asn Glu Lys Val
    50                  55                  60
Lys Gly Lys Val Thr Phe Thr Ala Asp Ala Ser Ser Asn Thr Ala Tyr
65                  70                  75                  80
Met Gln Leu Ser Ser Leu Thr Ser Glu Asp Ser Ala Val Tyr Tyr Cys
                85                  90                  95
Ala Arg Gly Asp Gly Phe Tyr Val Tyr Trp Gly Gln Gly Thr Thr Leu
            100                 105                 110
Thr Val Ser Ser Ala Ser Thr Lys Gly Pro Ser Val Phe Pro Leu Ala
        115                 120                 125
```

Pro Ser Ser Lys Ser Thr Ser Gly Gly Thr Ala Ala Leu Gly Cys Leu
130 135 140
Val Lys Asp Tyr Phe Pro Glu Pro Val Thr Val Ser Trp Asn Ser Gly
145 150 155 160
Ala Leu Thr Ser Gly Val His Thr Phe Pro Ala Val Leu Gln Ser Ser
165 170 175
Gly Leu Tyr Ser Leu Ser Ser Val Val Thr Val Pro Ser Ser Ser Leu
180 185 190
Gly Thr Gln Thr Tyr Ile Cys Asn Val Asn His Lys Pro Ser Asn Thr
195 200 205
Lys Val Asp Lys Lys Val Glu Pro Lys Ser Cys Asp Lys Thr His Thr
210 215 220
Cys Pro Pro Cys Pro Ala Pro Glu Leu Leu Gly Gly Pro Ser Val Phe
225 230 235 240
Leu Phe Pro Pro Lys Pro Lys Asp Thr Leu Met Ile Ser Arg Thr Pro
245 250 255
Glu Val Thr Cys Val Val Val Ala Val Ser His Glu Asp Pro Glu Val
260 265 270
Lys Phe Asn Trp Tyr Val Asp Gly Val Glu Val His Asn Ala Lys Thr
275 280 285
Lys Pro Arg Glu Glu Gln Tyr Asn Ser Thr Tyr Arg Val Val Ser Val
290 295 300
Leu Thr Val Leu His Gln Asp Trp Leu Asn Gly Lys Glu Tyr Lys Cys
305 310 315 320
Lys Val Ser Asn Lys Ala Leu Pro Ala Pro Ile Glu Lys Thr Ile Ser
325 330 335
Lys Ala Lys Gly Gln Pro Arg Glu Pro Gln Val Tyr Thr Leu Pro Pro
340 345 350
Ser Arg Asp Glu Leu Thr Lys Asn Gln Val Ser Leu Thr Cys Leu Val
355 360 365
Lys Gly Phe Tyr Pro Ser Asp Ile Ala Val Glu Trp Glu Ser Asn Gly
370 375 380
Gln Pro Glu Asn Asn Tyr Lys Thr Thr Pro Pro Val Leu Asp Ser Asp
385 390 395 400
Gly Ser Phe Phe Leu Tyr Ser Lys Leu Thr Val Asp Lys Ser Arg Trp
405 410 415
Gln Gln Gly Asn Val Phe Ser Cys Ser Val Met His Glu Ala Leu His
420 425 430
Asn His Tyr Thr Gln Lys Ser Leu Ser Leu Ser Pro Gly Ala Gly Gly
435 440 445
Gly Gly Ser Gly Gly Gly Gly Ser Gly Gly Gly Gly Ser Gly Gly Gly
450 455 460
Gly Ser Gly Ile Pro Pro His Val Gln Lys Ser Val Asn Asn Asp Met
465 470 475 480
Ile Val Thr Asp Asn Asn Gly Ala Val Lys Phe Pro Gln Leu Cys Lys
485 490 495
Phe Cys Asp Val Arg Phe Ser Thr Cys Asp Asn Gln Lys Ser Cys Met
500 505 510
Ser Asn Cys Ser Ile Thr Ser Ile Cys Glu Lys Pro Gln Glu Val Cys
515 520 525
Val Ala Val Trp Arg Lys Asn Asp Glu Asn Ile Thr Leu Glu Thr Val
530 535 540
Cys His Asp Pro Lys Leu Pro Tyr His Asp Phe Ile Leu Glu Asp Ala
545 550 555 560
Ala Ser Pro Lys Cys Ile Met Lys Glu Lys Lys Lys Pro Gly Glu Thr
565 570 575
Phe Phe Met Cys Ser Cys Ser Ser Asp Glu Cys Asn Asp Asn Ile Ile
580 585 590

Phe Ser Glu Glu Tyr Asn Thr Ser Asn Pro Asp
595 600

<210> 41
<211> 214
<212> PRT
<213> Synthetic sequence

<220>
<223> Light chain of IgG1-TGFβ RII

<400> 41
Asp Ile Glu Leu Thr Gln Ser Pro Ala Thr Leu Ser Val Thr Pro Gly
1 5 10 15
Asp Ser Val Ser Leu Ser Cys Arg Ala Ser Gln Ser Ile Ser Asn Asn
20 25 30
Leu His Trp Tyr Gln Gln Lys Ser His Glu Ser Pro Arg Leu Leu Ile
35 40 45
Lys Tyr Thr Ser Gln Ser Met Ser Gly Ile Pro Ser Arg Phe Ser Gly
50 55 60
Ser Gly Ser Gly Thr Asp Phe Thr Leu Ser Ile Asn Ser Val Glu Thr
65 70 75 80
Glu Asp Phe Gly Val Tyr Phe Cys Gln Gln Ser Gly Ser Trp Pro Arg
85 90 95
Thr Phe Gly Gly Gly Thr Lys Leu Asp Ile Lys Arg Thr Val Ala Ala
100 105 110
Pro Ser Val Phe Ile Phe Pro Pro Ser Asp Glu Gln Leu Lys Ser Gly
115 120 125
Thr Ala Ser Val Val Cys Leu Leu Asn Asn Phe Tyr Pro Arg Glu Ala
130 135 140
Lys Val Gln Trp Lys Val Asp Asn Ala Leu Gln Ser Gly Asn Ser Gln
145 150 155 160
Glu Ser Val Thr Glu Gln Asp Ser Lys Asp Ser Thr Tyr Ser Leu Ser
165 170 175
Ser Thr Leu Thr Leu Ser Lys Ala Asp Tyr Glu Lys His Lys Val Tyr
180 185 190
Ala Cys Glu Val Thr His Gln Gly Leu Ser Ser Pro Val Thr Lys Ser
195 200 205
Phe Asn Arg Gly Glu Cys
210

<210> 42
<211> 607
<212> PRT
<213> Artificial Sequence

<220>
<223> Heavy chain of M7824

<400> 42
Glu Val Gln Leu Leu Glu Ser Gly Gly Gly Leu Val Gln Pro Gly Gly
1 5 10 15
Ser Leu Arg Leu Ser Cys Ala Ala Ser Gly Phe Thr Phe Ser Ser Tyr
20 25 30
Ile Met Met Trp Val Arg Gln Ala Pro Gly Lys Gly Leu Glu Trp Val
35 40 45
Ser Ser Ile Tyr Pro Ser Gly Gly Ile Thr Phe Tyr Ala Asp Thr Val
50 55 60

Lys Gly Arg Phe Thr Ile Ser Arg Asp Asn Ser Lys Asn Thr Leu Tyr
65                  70                  75                  80
Leu Gln Met Asn Ser Leu Arg Ala Glu Asp Thr Ala Val Tyr Tyr Cys
                85                  90                  95
Ala Arg Ile Lys Leu Gly Thr Val Thr Thr Val Asp Tyr Trp Gly Gln
            100                 105                 110
Gly Thr Leu Val Thr Val Ser Ser Ala Ser Thr Lys Gly Pro Ser Val
        115                 120                 125
Phe Pro Leu Ala Pro Ser Ser Lys Ser Thr Ser Gly Gly Thr Ala Ala
    130                 135                 140
Leu Gly Cys Leu Val Lys Asp Tyr Phe Pro Glu Pro Val Thr Val Ser
145                 150                 155                 160
Trp Asn Ser Gly Ala Leu Thr Ser Gly Val His Thr Phe Pro Ala Val
                165                 170                 175
Leu Gln Ser Ser Gly Leu Tyr Ser Leu Ser Ser Val Val Thr Val Pro
            180                 185                 190
Ser Ser Ser Leu Gly Thr Gln Thr Tyr Ile Cys Asn Val Asn His Lys
        195                 200                 205
Pro Ser Asn Thr Lys Val Asp Lys Arg Val Glu Pro Lys Ser Cys Asp
    210                 215                 220
Lys Thr His Thr Cys Pro Pro Cys Pro Ala Pro Glu Leu Leu Gly Gly
225                 230                 235                 240
Pro Ser Val Phe Leu Phe Pro Pro Lys Pro Lys Asp Thr Leu Met Ile
                245                 250                 255
Ser Arg Thr Pro Glu Val Thr Cys Val Val Val Asp Val Ser His Glu
            260                 265                 270
Asp Pro Glu Val Lys Phe Asn Trp Tyr Val Asp Gly Val Glu Val His
        275                 280                 285
Asn Ala Lys Thr Lys Pro Arg Glu Glu Gln Tyr Asn Ser Thr Tyr Arg
    290                 295                 300
Val Val Ser Val Leu Thr Val Leu His Gln Asp Trp Leu Asn Gly Lys
305                 310                 315                 320
Glu Tyr Lys Cys Lys Val Ser Asn Lys Ala Leu Pro Ala Pro Ile Glu
                325                 330                 335
Lys Thr Ile Ser Lys Ala Lys Gly Gln Pro Arg Glu Pro Gln Val Tyr
            340                 345                 350
Thr Leu Pro Pro Ser Arg Glu Glu Met Thr Lys Asn Gln Val Ser Leu
        355                 360                 365
Thr Cys Leu Val Lys Gly Phe Tyr Pro Ser Asp Ile Ala Val Glu Trp
    370                 375                 380
Glu Ser Asn Gly Gln Pro Glu Asn Asn Tyr Lys Thr Thr Pro Pro Val
385                 390                 395                 400
Leu Asp Ser Asp Gly Ser Phe Phe Leu Tyr Ser Lys Leu Thr Val Asp
                405                 410                 415
Lys Ser Arg Trp Gln Gln Gly Asn Val Phe Ser Cys Ser Val Met His
            420                 425                 430
Glu Ala Leu His Asn His Tyr Thr Gln Lys Ser Leu Ser Leu Ser Pro
        435                 440                 445
Gly Ala Gly Gly Gly Gly Ser Gly Gly Gly Gly Ser Gly Gly Gly Gly
    450                 455                 460
Ser Gly Gly Gly Gly Ser Gly Ile Pro Pro His Val Gln Lys Ser Val
465                 470                 475                 480
Asn Asn Asp Met Ile Val Thr Asp Asn Asn Gly Ala Val Lys Phe Pro
                485                 490                 495
Gln Leu Cys Lys Phe Cys Asp Val Arg Phe Ser Thr Cys Asp Asn Gln
            500                 505                 510
Lys Ser Cys Met Ser Asn Cys Ser Ile Thr Ser Ile Cys Glu Lys Pro
        515                 520                 525

```
Gln Glu Val Cys Val Ala Val Trp Arg Lys Asn Asp Glu Asn Ile Thr
    530                 535                 540
Leu Glu Thr Val Cys His Asp Pro Lys Leu Pro Tyr His Asp Phe Ile
545                 550                 555                 560
Leu Glu Asp Ala Ala Ser Pro Lys Cys Ile Met Lys Glu Lys Lys Lys
                565                 570                 575
Pro Gly Glu Thr Phe Phe Met Cys Ser Cys Ser Ser Asp Glu Cys Asn
            580                 585                 590
Asp Asn Ile Ile Phe Ser Glu Glu Tyr Asn Thr Ser Asn Pro Asp
        595                 600                 605

<210>  43
<211>  216
<212>  PRT
<213>  Artificial Sequence

<220>
<223>  Light chain of M7824

<400>  43
Gln Ser Ala Leu Thr Gln Pro Ala Ser Val Ser Gly Ser Pro Gly Gln
1               5                   10                  15
Ser Ile Thr Ile Ser Cys Thr Gly Thr Ser Ser Asp Val Gly Gly Tyr
            20                  25                  30
Asn Tyr Val Ser Trp Tyr Gln Gln His Pro Gly Lys Ala Pro Lys Leu
        35                  40                  45
Met Ile Tyr Asp Val Ser Asn Arg Pro Ser Gly Val Ser Asn Arg Phe
    50                  55                  60
Ser Gly Ser Lys Ser Gly Asn Thr Ala Ser Leu Thr Ile Ser Gly Leu
65                  70                  75                  80
Gln Ala Glu Asp Glu Ala Asp Tyr Tyr Cys Ser Ser Tyr Thr Ser Ser
                85                  90                  95
Ser Thr Arg Val Phe Gly Thr Gly Thr Lys Val Thr Val Leu Gly Gln
            100                 105                 110
Pro Lys Ala Asn Pro Thr Val Thr Leu Phe Pro Pro Ser Ser Glu Glu
        115                 120                 125
Leu Gln Ala Asn Lys Ala Thr Leu Val Cys Leu Ile Ser Asp Phe Tyr
    130                 135                 140
Pro Gly Ala Val Thr Val Ala Trp Lys Ala Asp Gly Ser Pro Val Lys
145                 150                 155                 160
Ala Gly Val Glu Thr Thr Lys Pro Ser Lys Gln Ser Asn Asn Lys Tyr
                165                 170                 175
Ala Ala Ser Ser Tyr Leu Ser Leu Thr Pro Glu Gln Trp Lys Ser His
            180                 185                 190
Arg Ser Tyr Ser Cys Gln Val Thr His Glu Gly Ser Thr Val Glu Lys
        195                 200                 205
Thr Val Ala Pro Thr Glu Cys Ser
    210                 215
```